(19) Europäisches Patentamt
European Patent Office
Office européen des brevets

(11) EP 1 658 771 A1

# (12) EUROPEAN PATENT APPLICATION

published in accordance with Art. 158(3) EPC

(43) Date of publication:
**24.05.2006 Bulletin 2006/21**

(21) Application number: **04772036.2**

(22) Date of filing: **23.08.2004**

(51) Int Cl.:
***A01N 43/80*** *(1985.01)* ***C07D 275/06*** *(1985.01)*
***C07D 417/12*** *(1990.01)*

(86) International application number:
**PCT/JP2004/012075**

(87) International publication number:
**WO 2005/018327 (03.03.2005 Gazette 2005/09)**

(84) Designated Contracting States:
**AT BE BG CH CY CZ DE DK EE ES FI FR GB GR HU IE IT LI LU MC NL PL PT RO SE SI SK TR**

(30) Priority: **26.08.2003 JP 2003300797**

(71) Applicants:
- **KUMIAI CHEMICAL INDUSTRY CO., LTD.**
  **Tokyo 110-0008 (JP)**
- **IHARA CHEMICAL INDUSTRY CO., LTD.**
  **Taitoh-ku,**
  **Tokyo 110-0008 (JP)**

(72) Inventors:
- **ITSUKI, Yoshinori,**
  **K.I Chem. Res. Inst. Co. Ltd.**
  **Iwata-gun,**
  **Shizuoka 4371213 (JP)**
- **SHIBATA, Masaru,**
  **K.I Chem. Research Inst. Co. Ltd.**
  **Iwata-gun,**
  **Shizuoka 4371213 (JP)**
- **KAJIKI, Ryu,**
  **c/o K.I Chem. Research Inst. Co. Ltd.**
  **Iwata-gun,**
  **Shizuoka 4371213 (JP)**
- **FUKUMOTO, Shunichirou,**
  **K.I Chem. Res. Inst. Co Ltd**
  **Iwata-gun,**
  **Shizuoka 4371213 (JP)**
- **FURUSE, Katsumi,**
  **Kumiai Chem. Industry Co. Ltd.**
  **Taito-ku,**
  **Tokyo 1100008 (JP)**
- **YAMAJI, Kouji,**
  **Kumiai Chemical Industry Co. Ltd.**
  **Taito-ku,**
  **Tokyo 1100008 (JP)**
- **TAKAHASHI, Satoru,**
  **Kumiai Chemical Ind. Co. Ltd.**
  **Taito-ku,**
  **Tokyo 1100008 (JP)**
- **ITOU, Yoshihiro,**
  **Kumiai Chemical Industry Co. Ltd.**
  **Taito-ku,**
  **Tokyo 1100008 (JP)**

(74) Representative: **Zinnecker, Armin et al**
**Lorenz-Seidler-Gossel,**
**Widenmayerstrasse 23**
**80538 München (DE)**

## (54) BENZISOTHIAZOLINE DERIVATIVE, PLANT DISEASE CONTROL AGENT FOR AGRICULTURAL OR HORTICULTURAL USE, AND PEST CONTROL AGENT FOR AGRICULTURAL OR HORTICULTURAL USE

(57) A plant disease control agent for agricultural or horticultural use and a pest control agent for agricultural or horticultural use, which contain a benzisothiazoline derivative or a salt thereof as an active ingredient and have the following marked effects. They are highly effective in controlling agricultural or horticultural plant diseases and are highly effective in controlling a wide range of pests. They are effective also in controlling pests having resistance. Furthermore, they are excellent in residual activity and rain resistance without causing chemical damage to crops.

The benzisothiazoline derivative of the present invention is represented by the following general formula [I]. The symbols in the formula are defined in the Description.

EP 1 658 771 A1

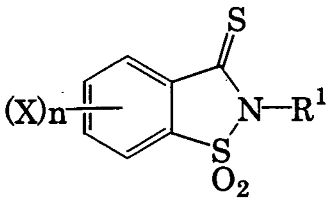

[ I ]

## Description

Technical Field

The present invention relates to a benzisothiazoline derivative or a salt thereof, as well as to a plant disease control agent for agricultural or horticultural use or a pest control agent for agricultural or horticultural use both containing such a derivative or a salt thereof as an active ingredient. More particularly, the present invention relates to a particular benzisothiazoline derivative and a method for its use.

Background Art

As to the compounds belonging to benzisothiazoline derivatives, there are known those described in the following Patent Literatures 1 to 3. In the Patent Literatures 1 and 3, there are descriptions on medicines and, in the Patent Literature 2, there is a description on raw materials for heat-resistant polymer. In any of these Patent Literatures, there is no description on plant disease control agent for agricultural or horticultural use or on pest control agent for agricultural or horticultural use.

Patent Literature 1: EP-81955
Patent Literature 2: EP-61434
Patent Literature 3: RU-2039052

In agricultural and horticultural fields, various synthetic agricultural chemicals have been used for plant disease control or pest control because the diseases caused by plant pathogenic fungi and the damages brought about by pests give a serious influence on the production efficiency of agriculture.

However, plant disease control agents for agricultural or horticultural use, which have been used widely, are not fully satisfactory in effect, spectrum, residual activity, etc. Moreover, in recent years, there have arisen problems such as reduction in chemical activity owing to the emergence of resistant fungi. Hence, it is desired to develop a safer plant disease control agent for agricultural or horticultural use.

Further, among the existing pest control agents for agricultural or horticultural use, there are those whose use is regulated for problems such as residue, accumulation, environmental pollution and the like, or those which have a reduced activity because long-term use thereof have invited the emergence of resistant pests.

Recently, requirements for the safety of chemical substances as well as for the influences of these substances on environment have become higher. Therefore, it is desired to develop a plant disease control agent and a pest control agent, which are highly effective at a low dosage and excellent in safety.

In order to achieve the above task, the present inventors synthesized a number of benzisothiazoline derivatives whose plant disease control activity and pest control activity have been unknown, and investigated their activities for plant disease control and pest control and their utilities. As a result, it was found that a benzisothiazoline derivative represented by the following general formula [I] or a salt thereof (hereinafter, the derivative or salt is referred to as the present application compound) is a compound having the above-mentioned feature. The finding has led to the completion of the present invention. The present invention provides the followings.

(1) A plant disease control agent for agricultural or horticultural use, containing, as an active ingredient, a benzisothiazoline derivative represented by the following general formula [I] or a salt thereof:

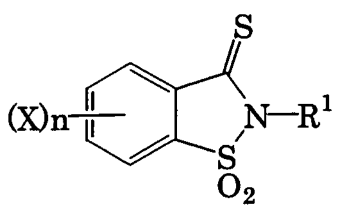

[I]

{wherein $R^1$ is $C_1$-$C_{12}$ alkyl group, $C_2$-$C_6$ alkenyl group, $C_2$-$C_6$ alkynyl group, $C_1$-$C_6$ alkoxy group, $C_3$-$C_6$ cycloalkyl group, $C_1$-$C_6$ haloalkyl group, $C_2$-$C_6$ haloalkenyl group, $C_3$-$C_6$ cycloalkyl $C_1$-$C_6$ alkyl group, $C_1$-$C_6$ alkoxy $C_1$-$C_6$ alkyl group, $C_1$-$C_6$ alkylthio $C_1$-$C_6$ alkyl group, -C(=Y)-$R^2$ group, -C(=Y)-$Y^1$-$R^3$ group, -W-C(=O)-$R^4$ group, -W-(C(=O)-$R^4$)$_2$ group, -W-$Y^1$-W-$R^{12}$ group, -W-$Y^1$-(C(=Y))$_m$-$R^{12}$ group, -W-$R^{12}$ group, -$Y^1$-W-$R^{12}$ group, -W-$Y^1$-N=C$R^{12}R^{14}$ group, hydroxy $C_1$-$C_6$ alkyl group, cyano $C_1$-$C_6$ alkyl group, cyanothio $C_1$-$C_6$ alkyl group, isothiocyano

$C_1$-$C_6$ alkyl group, nitro $C_1$-$C_6$ alkyl group, $NR^6R^7$ $C_1$-$C_6$ alkyl group, phenyl $C_1$-$C_6$ alkyl group (this phenyl $C_1$-$C_6$ alkyl group may be substituted with halogen atom, nitro group, cyano group, $C_1$-$C_6$ alkyl group, $C_1$-$C_6$ alkoxy group, $C_1$-$C_6$ haloalkyl group, $C_1$-$C_6$ haloalkoxy group or $NR^6R^7$ group), phenyl group (this phenyl group may be substituted with halogen atom, nitro group, cyano group, $C_1$-$C_6$ alkyl group, $C_1$-$C_6$ alkoxy group, $C_1$-$C_6$ haloalkyl group, $C_1$-$C_6$ haloalkoxy group or $NR^6R^7$ group), or $C_3$-$C_{10}$ heterocyclic group having at least one hetero atom which is selected from oxygen atom, sulfur atom and nitrogen atom and which may be the same or different from each other (this heterocyclic group may be substituted with halogen atom, nitro group, cyano group, $C_1$-$C_6$ alkyl group, $C_1$-$C_6$ alkoxy group, $C_1$-$C_6$ haloalkyl group, $C_1$-$C_6$ haloalkoxy group or $NR^6R^7$ group); X is halogen atom, nitro group, cyano group, $C_1$-$C_6$ alkyl group, $C_1$-$C_6$ alkoxy group, $C_1$-$C_6$ haloalkyl group, $C_1$-$C_6$ haloalkoxy group, or $NR^6R^7$ group;

$R^2$ is $C_1$-$C_6$ alkyl group, $C_1$-$C_6$ alkenyl group, -W-$R^{12}$ group, $C_1$-$C_6$ haloalkyl group, $NR^{15}R^{16}$ group, or phenyl group (this phenyl group may be substituted with halogen atom, nitro group, cyano group, $C_1$-$C_6$ alkyl group, $C_1$-$C_6$ alkoxy group, $C_1$-$C_6$ haloalkyl group, $C_1$-$C_6$ haloalkoxy group or $NR^8R^9$ group);

$R^3$ is $C_1$-$C_{12}$ alkyl group, $C_2$-$C_6$ alkenyl group, $C_2$-$C_6$ alkynyl group, $C_3$-$C_6$ cycloalkyl group, $C_1$-$C_6$ haloalkyl group, $C_1$-$C_6$ alkoxy $C_1$-$C_6$ alkyl group, -W-$R^{12}$ group, benzyl group (this benzyl group may be substituted with halogen atom, nitro group, cyano group, $C_1$-$C_6$ alkyl group, $C_1$-$C_6$ alkoxy group, $C_1$-$C_6$ haloalkyl group, $C_1$-$C_6$ haloalkoxy group or $NR^8R^9$ group), or phenyl group (this phenyl group may be substituted with halogen atom, nitro group, cyano group, $C_1$-$C_6$ alkyl group, $C_1$-$C_6$ alkoxy group, $C_1$-$C_6$ haloalkyl group, $C_1$-$C_6$ haloalkoxy group or $NR^8R^9$ group);

$R^4$ is $C_1$-$C_6$ alkyl group, $C_1$-$C_6$ haloalkyl group, phenyl group (this phenyl group may be substituted with halogen atom, nitro group, cyano group, $C_1$-$C_6$ alkyl group, $C_1$-$C_6$ alkoxy group, $C_1$-$C_6$ haloalkyl group, $C_1$-$C_6$ haloalkoxy group or $NR^8R^9$ group), $NR^6R^7$ group or $Y^2R^5$ group ($Y^2$ is oxygen atom or sulfur atom); $R^5$ is $C_1$-$C_{12}$ alkyl group, $C_2$-$C_6$ alkenyl group, $C_2$-$C_6$ alkynyl group, $C_3$-$C_6$ cycloalkyl group, $C_1$-$C_6$ haloalkyl group, $C_1$-$C_6$ alkoxy $C_1$-$C_6$ alkyl group, benzyl group (this benzyl group may be substituted with halogen atom, nitro group, cyano group, $C_1$-$C_6$ alkyl group, $C_1$-$C_6$ alkoxy group, $C_1$-$C_6$ haloalkyl group, $C_1$-$C_6$ haloalkoxy group or $NR^8R^9$ group), or phenyl group (this phenyl group may be substituted with halogen atom, nitro group, cyano group, $C_1$-$C_6$ alkyl group, $C_1$-$C_6$ alkoxy group, $C_1$-$C_6$ haloalkyl group, $C_1$-$C_6$ haloalkoxy group or $NR^8R^9$ group);

$R^6$ and $R^7$ are each independently hydrogen atom, $C_1$-$C_6$ alkyl group, $C_2$-$C_6$ alkenyl group, -W-$R^{12}$ group, or phenyl group (this phenyl group may be substituted with halogen atom, nitro group, cyano group, $C_1$-$C_6$ alkyl group, $C_1$-$C_6$ alkoxy group, $C_1$-$C_6$ haloalkyl group, $C_1$-$C_6$ haloalkoxy group or $NR^8R^9$ group);

$R^8$ and $R^9$ are each independently hydrogen atom or $C_1$-$C_6$ alkyl group;

$R^{12}$ is phenyl group (this phenyl group may be substituted with halogen atom, nitro group, cyano group, $C_1$-$C_6$ alkyl group, $C_1$-$C_6$ alkoxy group, $C_1$-$C_6$ haloalkyl group, $C_1$-$C_6$ haloalkoxy group or $NR^8R^9$ group), or $C_3$-$C_{10}$ heterocyclic group having at least one hetero atom which is selected from oxygen atom, sulfur atom and nitrogen atom and which may be the same or different from each other (this heterocyclic group may be substituted with halogen atom, nitro group, cyano group, $C_1$-$C_6$ alkyl group, $C_1$-$C_6$ alkoxy group, $C_1$-$C_6$ haloalkyl group, $C_1$-$C_6$ haloalkoxy group or $NR^6R^7$ group);

$R^{13}$ is $C_3$-$C_{10}$ heterocyclic group having at least one hetero atom which is selected from oxygen atom, sulfur atom and nitrogen atom and which may be the same or different from each other (this heterocyclic group may be substituted with halogen atom, nitro group, cyano group, $C_1$-$C_6$ alkyl group, $C_1$-$C_6$ alkoxy group, $C_1$-$C_6$ haloalkyl group, $C_1$-$C_6$ haloalkoxy group or $NR^6R^7$ group);

$R^{14}$ is hydrogen atom or $C_1$-$C_6$ alkyl group;

$R^{15}$ and $R^{16}$ are each independently $C_1$-$C_6$ alkyl group or -W-$R^{13}$ group (when either one of $R^{15}$ and $R^{16}$ is -W-$R^{13}$ group, the other may be hydrogen atom);

n is an integer of 0 to 4;

m is an integer of 0 or 1;

Y is oxygen atom or sulfur atom;

$Y^1$ is oxygen atom or sulfur atom; and

W is $C_1$-$C_4$ alkylene group or $C_1$-$C_4$ alkenylene group}.

(2) A pest control agent for agricultural or horticultural use, containing, as an active ingredient, a benzisothiazoline derivative represented by the following general formula [I] or a salt thereof:

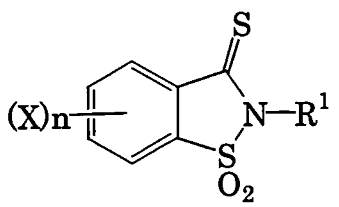

[ I ]

{wherein $R^1$ is $C_1$-$C_{12}$ alkyl group, $C_2$-$C_6$ alkenyl group, $C_2$-$C_6$ alkynyl group, $C_1$-$C_6$ alkoxy group, $C_3$-$C_6$ cycloalkyl group, $C_1$-$C_6$ haloalkyl group, $C_2$-$C_6$ haloalkenyl group, $C_3$-$C_6$ cycloalkyl $C_1$-$C_6$ alkyl group, $C_1$-$C_6$ alkoxy $C_1$-$C_6$ alkyl group, $C_1$-$C_6$ alkylthio $C_1$-$C_6$ alkyl group, -C(=Y)-$R^2$ group, -C(=Y)-$Y^1$-$R^3$ group, -W-C(=O)-$R^4$ group, -W-(C(=O)-$R^4$)$_2$ group, -W-$Y^1$-W-$R^{12}$ group, -W-$Y^1$-(C(=Y))$_m$-$R^{12}$ group, -W-$R^{12}$ group, -$Y^1$-W-$R^{12}$ group, -W-$Y^1$-N=C$R^{12}R^{14}$ group, hydroxy $C_1$-$C_6$ alkyl group, cyano $C_1$-$C_6$ alkyl group, cyanothio $C_1$-$C_6$ alkyl group, isothiocyano $C_1$-$C_6$ alkyl group, nitro $C_1$-$C_6$ alkyl group, $NR^6R^7$ $C_1$-$C_6$ alkyl group, phenyl $C_1$-$C_6$ alkyl group (this phenyl $C_1$-$C_6$ alkyl group may be substituted with halogen atom, nitro group, cyano group, $C_1$-$C_6$ alkyl group, $C_1$-$C_6$ alkoxy group, $C_1$-$C_6$ haloalkyl group, $C_1$-$C_6$ haloalkoxy group or $NR^6R^7$ group), phenyl group (this phenyl group may be substituted with halogen atom, nitro group, cyano group, $C_1$-$C_6$ alkyl group, $C_1$-$C_6$ alkoxy group, $C_1$-$C_6$ haloalkyl group, $C_1$-$C_6$ haloalkoxy group or $NR^6R^7$ group), or $C_3$-$C_{10}$ heterocyclic group having at least one hetero atom which is selected from oxygen atom, sulfur atom and nitrogen atom and which may be the same or different from each other (this heterocyclic group may be substituted with halogen atom, nitro group, cyano group, $C_1$-$C_6$ alkyl group, $C_1$-$C_6$ alkoxy group, $C_1$-$C_6$ haloalkyl group, $C_1$-$C_6$ haloalkoxy group or $NR^6R^7$ group); X is halogen atom, nitro group, cyano group, $C_1$-$C_6$ alkyl group, $C_1$-$C_6$ alkoxy group, $C_1$-$C_6$ haloalkyl group, $C_1$-$C_6$ haloalkoxy group, or $NR^6R^7$ group;

$R^2$ is $C_1$-$C_6$ alkyl group, $C_1$-$C_6$ alkenyl group, -W-$R^{12}$ group, $C_1$-$C_6$ haloalkyl group, $NR^{15}R^{16}$ group, or phenyl group (this phenyl group may be substituted with halogen atom, nitro group, cyano group, $C_1$-$C_6$ alkyl group, $C_1$-$C_6$ alkoxy group, $C_1$-$C_6$ haloalkyl group, $C_1$-$C_6$ haloalkoxy group or $NR^8R^9$ group);

$R^3$ is $C_1$-$C_{12}$ alkyl group, $C_2$-$C_6$ alkenyl group, $C_2$-$C_6$ alkynyl group, $C_3$-$C_6$ cycloalkyl group, $C_1$-$C_6$ haloalkyl group, $C_1$-$C_6$ alkoxy $C_1$-$C_6$ alkyl group, -W-$R^{12}$ group, benzyl group (this benzyl group may be substituted with halogen atom, nitro group, cyano group, $C_1$-$C_6$ alkyl group, $C_1$-$C_6$ alkoxy group, $C_1$-$C_6$ haloalkyl group, $C_1$-$C_6$ haloalkoxy group or $NR^8R^9$ group), or phenyl group (this phenyl group may be substituted with halogen atom, nitro group, cyano group, $C_1$-$C_6$ alkyl group, $C_1$-$C_6$ alkoxy group, $C_1$-$C_6$ haloalkyl group, $C_1$-$C_6$ haloalkoxy group or $NR^8R^9$ group);

$R^4$ is $C_1$-$C_6$ alkyl group, $C_1$-$C_6$ haloalkyl group, phenyl group (this phenyl group may be substituted with halogen atom, nitro group, cyano group, $C_1$-$C_6$ alkyl group, $C_1$-$C_6$ alkoxy group, $C_1$-$C_6$ haloalkyl group, $C_1$-$C_6$ haloalkoxy group or $NR^8R^9$ group), $NR^6R^7$ group or $Y^2R^5$ group ($Y^2$ is oxygen atom or sulfur atom); $R^5$ is $C_1$-$C_{12}$ alkyl group, $C_2$-$C_6$ alkenyl group, $C_2$-$C_6$ alkynyl group, $C_3$-$C_6$ cycloalkyl group, $C_1$-$C_6$ haloalkyl group, $C_1$-$C_6$ alkoxy $C_1$-$C_6$ alkyl group, benzyl group (this benzyl group may be substituted with halogen atom, nitro group, cyano group, $C_1$-$C_6$ alkyl group, $C_1$-$C_6$ alkoxy group, $C_1$-$C_6$ haloalkyl group, $C_1$-$C_6$ haloalkoxy group or $NR^8R^9$ group), or phenyl group (this phenyl group may be substituted with halogen atom, nitro group, cyano group, $C_1$-$C_6$ alkyl group, $C_1$-$C_6$ alkoxy group, $C_1$-$C_6$ haloalkyl group, $C_1$-$C_6$ haloalkoxy group or $NR^8R^9$ group);

$R^6$ and $R^7$ are each independently hydrogen atom, $C_1$-$C_6$ alkyl group, $C_2$-$C_6$ alkenyl group, -W-$R^{12}$ group, or phenyl group (this phenyl group may be substituted with halogen atom, nitro group, cyano group, $C_1$-$C_6$ alkyl group, $C_1$-$C_6$ alkoxy group, $C_1$-$C_6$ haloalkyl group, $C_1$-$C_6$ haloalkoxy group or $NR^8R^9$ group);

$R^8$ and $R^9$ are each independently hydrogen atom or $C_1$-$C_6$ alkyl group;

$R^{12}$ is phenyl group (this phenyl group may be substituted with halogen atom, nitro group, cyano group, $C_1$-$C_6$ alkyl group, $C_1$-$C_6$ alkoxy group, $C_1$-$C_6$ haloalkyl group, $C_1$-$C_6$ haloalkoxy group or $NR^8R^9$ group), or $C_3$-$C_{10}$ heterocyclic group having at least one hetero atom which is selected from oxygen atom, sulfur atom and nitrogen atom and which may be the same or different from each other (this heterocyclic group may be substituted with halogen atom, nitro group, cyano group, $C_1$-$C_6$ alkyl group, $C_1$-$C_6$ alkoxy group, $C_1$-$C_6$ haloalkyl group, $C_1$-$C_6$ haloalkoxy group or $NR^6R^7$ group);

$R^{13}$ is $C_3$-$C_{10}$ heterocyclic group having at least one hetero atom which is selected from oxygen atom, sulfur atom and nitrogen atom and which may be the same or different from each other (this heterocyclic group may be substituted with halogen atom, nitro group, cyano group, $C_1$-$C_6$ alkyl group, $C_1$-$C_6$ alkoxy group, $C_1$-$C_6$ haloalkyl group, $C_1$-$C_6$ haloalkoxy group or $NR^6R^7$ group);

$R^{14}$ is hydrogen atom or $C_1$-$C_6$ alkyl group;

$R^{15}$ and $R^{16}$ are each independently $C_1$-$C_6$ alkyl group or -W-$R^{13}$ group (when either one of $R^{15}$ and $R^{16}$ is -W-$R^{13}$ group, the other may be hydrogen atom);

n is an integer of 0 to 4;

m is an integer of 0 or 1;

Y is oxygen atom or sulfur atom;

$Y^1$ is oxygen atom or sulfur atom; and

W is $C_1$-$C_4$ alkylene group or $C_1$-$C_4$ alkenylene group}.

(3) A benzisothiazoline derivative represented by the following general formula [I] or a salt thereof:

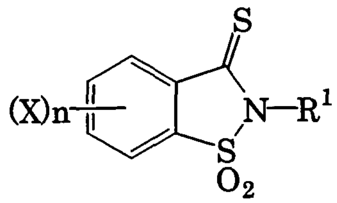

[I]

{wherein $R^1$ is $C_3$-$C_{12}$ alkyl group, $C_2$-$C_6$ alkenyl group, $C_2$-$C_6$ alkynyl group, $C_1$-$C_6$ alkoxy group, $C_3$-$C_6$ cycloalkyl group, $C_1$-$C_6$ haloalkyl group, $C_2$-$C_6$ haloalkenyl group, $C_3$-$C_6$ cycloalkyl $C_1$-$C_6$ alkyl group, $C_1$-$C_6$ alkoxy $C_1$-$C_6$ alkyl group, $C_1$-$C_6$ alkylthio $C_1$-$C_6$ alkyl group, -C(=Y)-$R^2$ group, -C(=Y)-$Y^1$-$R^3$ group, -W-C(=O)-$R^4$ group, -W-(C(=O)-$R^4$)$_2$ group, -W-$Y^1$-W-$R^{12}$ group, -W-$Y^1$-(C(=Y))$_m$-$R^{12}$ group, -W-$R^{13}$ group, -$Y^1$-W-$R^{12}$ group, -W-$Y^1$-N=$CR^{12}R^{14}$ group, hydroxy $C_1$-$C_6$ alkyl group, cyano $C_1$-$C_6$ alkyl group, cyanothio $C_1$-$C_6$ alkyl group, isothiocyano $C_1$-$C_6$ alkyl group, nitro $C_1$-$C_6$ alkyl group, $NR^6R^7$ $C_1$-$C_6$ alkyl group, phenyl group (this phenyl group may be substituted with halogen atom, nitro group, cyano group, $C_1$-$C_6$ alkyl group, $C_1$-$C_6$ alkoxy group, $C_1$-$C_6$ haloalkyl group, $C_1$-$C_6$ haloalkoxy group or $NR^6R^7$ group), or $C_3$-$C_{10}$ heterocyclic group having at least one hetero atom which is selected from oxygen atom, sulfur atom and nitrogen atom and which may be the same or different from each other (this heterocyclic group may be substituted with halogen atom, nitro group, cyano group, $C_1$-$C_6$ alkyl group, $C_1$-$C_6$ alkoxy group, $C_1$-$C_6$ haloalkyl group, $C_1$-$C_6$ haloalkoxy group or $NR^6R^7$ group);

X is halogen atom, nitro group, cyano group, $C_1$-$C_6$ alkyl group, $C_1$-$C_6$ alkoxy group, $C_1$-$C_6$ haloalkyl group, $C_1$-$C_6$ haloalkoxy group, or $NR^6R^7$ group;

$R^2$ is $C_1$-$C_6$ alkyl group, $C_1$-$C_6$ alkenyl group, -W-$R^{12}$ group, $C_1$-$C_6$ haloalkyl group, $NR^{15}R^{16}$ group, or phenyl group (this phenyl group may be substituted with halogen atom, nitro group, cyano group, $C_1$-$C_6$ alkyl group, $C_1$-$C_6$ alkoxy group, $C_1$-$C_6$ haloalkyl group, $C_1$-$C_6$ haloalkoxy group or $NR^8R^9$ group);

$R^3$ is $C_1$-$C_{12}$ alkyl group, $C_2$-$C_6$ alkenyl group, $C_2$-$C_6$ alkynyl group, $C_3$-$C_6$ cycloalkyl group, $C_1$-$C_6$ haloalkyl group, $C_1$-$C_6$ alkoxy $C_1$-$C_6$ alkyl group, -W-$R^{12}$ group, benzyl group (this benzyl group may be substituted with halogen atom, nitro group, cyano group, $C_1$-$C_6$ alkyl group, $C_1$-$C_6$ alkoxy group, $C_1$-$C_6$ haloalkyl group, $C_1$-$C_6$ haloalkoxy group or $NR^8R^9$ group), or phenyl group (this phenyl group may be substituted with halogen atom, nitro group, cyano group, $C_1$-$C_6$ alkyl group, $C_1$-$C_6$ alkoxy group, $C_1$-$C_6$ haloalkyl group, $C_1$-$C_6$ haloalkoxy group or $NR^8R^9$ group);

$R^4$ is $C_1$-$C_6$ alkyl group, $C_1$-$C_6$ haloalkyl group, phenyl group (this phenyl group may be substituted with halogen atom, nitro group, cyano group, $C_1$-$C_6$ alkyl group, $C_1$-$C_6$ alkoxy group, $C_1$-$C_6$ haloalkyl group, $C_1$-$C_6$ haloalkoxy group or $NR^8R^9$ group), $NR^6R^7$ group or $Y^2R^5$ group ($Y^2$ is oxygen atom or sulfur atom);

$R^5$ is $C_2$-$C_6$ alkenyl group, $C_2$-$C_6$ alkynyl group, $C_3$-$C_6$ cycloalkyl group, $C_1$-$C_6$ haloalkyl group, $C_1$-$C_6$ alkoxy $C_1$-$C_6$ alkyl group, benzyl group (this benzyl group may be substituted with halogen atom, nitro group, cyano group, $C_1$-$C_6$ alkyl group, $C_1$-$C_6$ alkoxy group, $C_1$-$C_6$ haloalkyl group, $C_1$-$C_6$ haloalkoxy group or $NR^8R^9$ group), or phenyl group (this phenyl group may be substituted with halogen atom, nitro group, cyano group, $C_1$-$C_6$ alkyl group, $C_1$-$C_6$ alkoxy group, $C_1$-$C_6$ haloalkyl group, $C_1$-$C_6$ haloalkoxy group or $NR^8R^9$ group);

$R^6$ and $R^7$ are each independently hydrogen atom, $C_1$-$C_6$ alkyl group, $C_2$-$C_6$ alkenyl group, -W-$R^{12}$ group, or phenyl group (this phenyl group may be substituted with halogen atom, nitro group, cyano group, $C_1$-$C_6$ alkyl group, $C_1$-$C_6$ alkoxy group, $C_1$-$C_6$ haloalkyl group, $C_1$-$C_6$ haloalkoxy group or $NR^8R^9$ group);

$R^8$ and $R^9$ are each independently hydrogen atom or $C_1$-$C_6$ alkyl group;

$R^{12}$ is phenyl group (this phenyl group may be substituted with halogen atom, nitro group, cyano group, $C_1$-$C_6$ alkyl group, $C_1$-$C_6$ alkoxy group, $C_1$-$C_6$ haloalkyl group, $C_1$-$C_6$ haloalkoxy group or $NR^8R^9$ group), or $C_3$-$C_{10}$ heterocyclic group having at least one hetero atom which is selected from oxygen atom, sulfur atom and nitrogen atom and which may be the same or different from each other (this heterocyclic group may be substituted with halogen atom, nitro group, cyano group, $C_1$-$C_6$ alkyl group, $C_1$-$C_6$ alkoxy group, $C_1$-$C_6$ haloalkyl group, $C_1$-$C_6$ haloalkoxy group or $NR^6R^7$ group);

$R^{13}$ is $C_3$-$C_{10}$ heterocyclic group having at least one hetero atom which is selected from oxygen atom, sulfur atom and nitrogen atom and which may be the same or different from each other (this heterocyclic group may be substituted with halogen atom, nitro group, cyano group, $C_1$-$C_6$ alkyl group, $C_1$-$C_6$ alkoxy group, $C_1$-$C_6$ haloalkyl group, $C_1$-$C_6$ haloalkoxy group or $NR^6R^7$ group);

$R^{14}$ is hydrogen atom or $C_1$-$C_6$ alkyl group;

$R^{15}$ and $R^{16}$ are each independently $C_1$-$C_6$ alkyl group or -W-$R^{13}$ group (when either one of $R^{15}$ and $R^{16}$ is -W-$R^{13}$ group, the other may be hydrogen atom);

n is an integer of 0 to 4;

m is an integer of 0 or 1;

Y is oxygen atom or sulfur atom;

$Y^1$ is oxygen atom or sulfur atom; and
W is $C_1$-$C_4$ alkylene group or $C_1$-$C_4$ alkenylene group}.
(4) A benzisothiazoline derivative represented by the following general formula [I] or a salt thereof:

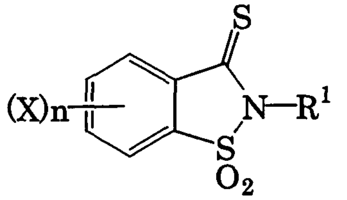

[I]

{wherein $R^1$ is $C_2$-$C_6$ alkenyl group, $C_2$-$C_6$ alkynyl group, $C_1$-$C_6$ alkoxy group, $C_3$-$C_6$ cycloalkyl group, $C_1$-$C_6$ haloalkyl group, $C_2$-$C_6$ haloalkenyl group, $C_3$-$C_6$ cycloalkyl $C_1$-$C_6$ alkyl group, $C_1$-$C_6$ alkoxy $C_1$-$C_6$ alkyl group, $C_1$-$C_6$ alkylthio $C_1$-$C_6$ alkyl group, -C(=Y)-$R^2$ group, -C (=Y)-$Y^1$-$R^3$ group, -W-C(=O)-$R^4$ group, -W-(C(=O)-$R^4$)$_2$ group, -W-$Y^1$-W-$R^{12}$ group, -W-$Y^1$-(C(=Y))$_m$-$R^{12}$ group, -W-$R^{13}$ group, -$Y^1$-W-$R^{12}$ group, -W-$Y^1$-N=C$R^{12}R^{14}$ group, hydroxy $C_1$-$C_6$ alkyl group, cyano $C_1$-$C_6$ alkyl group, cyanothio $C_1$-$C_6$ alkyl group, isothiocyano $C_1$-$C_6$ alkyl group, nitro $C_1$-$C_6$ alkyl group, $NR^6R^7$ $C_1$-$C_6$ alkyl group, phenyl group (this phenyl group may be substituted with halogen atom, nitro group, cyano group, $C_1$-$C_6$ alkyl group, $C_1$-$C_6$ alkoxy group, $C_1$-$C_6$ haloalkyl group, $C_1$-$C_6$ haloalkoxy group or $NR^6R^7$ group), or $C_3$-$C_{10}$ heterocyclic group having at least one hetero atom which is selected from oxygen atom, sulfur atom and nitrogen atom and which may be the same or different from each other (this heterocyclic group may be substituted with halogen atom, nitro group, cyano group, $C_1$-$C_6$ alkyl group, $C_1$-$C_6$ alkoxy group, $C_1$-$C_6$ haloalkyl group, $C_1$-$C_6$ haloalkoxy group or $NR^6R^7$ group);
X is halogen atom, nitro group, cyano group, $C_1$-$C_6$ alkyl group, $C_1$-$C_6$ alkoxy group, $C_1$-$C_6$ haloalkyl group, $C_1$-$C_6$ haloalkoxy group, or $NR^6R^7$ group;
$R^2$ is $C_1$-$C_6$ alkyl group, $C_1$-$C_6$ alkenyl group, -W-$R^{12}$ group, $C_1$-$C_6$ haloalkyl group, or phenyl group (this phenyl group may be substituted with halogen atom, nitro group, cyano group, $C_1$-$C_6$ alkyl group, $C_1$-$C_6$ alkoxy group, $C_1$-$C_6$ haloalkyl group, $C_1$-$C_6$ haloalkoxy group or $NR^8R^9$ group);
$R^3$ is $C_1$-$C_{12}$ alkyl group, $C_2$-$C_6$ alkenyl group, $C_2$-$C_6$ alkynyl group, $C_3$-$C_6$ cycloalkyl group, $C_1$-$C_6$ haloalkyl group, $C_1$-$C_6$ alkoxy $C_1$-$C_6$ alkyl group, -W-$R^{12}$ group, benzyl group (this benzyl group may be substituted with halogen atom, nitro group, cyano group, $C_1$-$C_6$ alkyl group, $C_1$-$C_6$ alkoxy group, $C_1$-$C_6$ haloalkyl group, $C_1$-$C_6$ haloalkoxy group or $NR^8R^9$ group), or phenyl group (this phenyl group may be substituted with halogen atom, nitro group, cyano group, $C_1$-$C_6$ alkyl group, $C_1$-$C_6$ alkoxy group, $C_1$-$C_6$ haloalkyl group, $C_1$-$C_6$ haloalkoxy group or $NR^8R^9$ group);
$R^4$ is $C_1$-$C_6$ alkyl group, $C_1$-$C_6$ haloalkyl group, phenyl group (this phenyl group may be substituted with halogen atom, nitro group, cyano group, $C_1$-$C_6$ alkyl group, $C_1$-$C_6$ alkoxy group, $C_1$-$C_6$ haloalkyl group, $C_1$-$C_6$ haloalkoxy group or $NR^8R^9$ group), $NR^6R^7$ group or $Y^2R^5$ group ($Y^2$ is oxygen atom or sulfur atom);
$R^5$ is $C_2$-$C_6$ alkenyl group, $C_2$-$C_6$ alkynyl group, $C_3$-$C_6$ cycloalkyl group, $C_1$-$C_6$ haloalkyl group, $C_1$-$C_6$ alkoxy $C_1$-$C_6$ alkyl group, benzyl group (this benzyl group may be substituted with halogen atom, nitro group, cyano group, $C_1$-$C_6$ alkyl group, $C_1$-$C_6$ alkoxy group, $C_1$-$C_6$ haloalkyl group, $C_1$-$C_6$ haloalkoxy group or $NR^8R^9$ group), or phenyl group (this phenyl group may be substituted with halogen atom, nitro group, cyano group, $C_1$-$C_6$ alkyl group, $C_1$-$C_6$ alkoxy group, $C_1$-$C_6$ haloalkyl group, $C_1$-$C_6$ haloalkoxy group or $NR^8R^9$ group);
$R^6$ and $R^7$ are each independently hydrogen atom, $C_1$-$C_6$ alkyl group, $C_2$-$C_6$ alkenyl group, -W-$R^{12}$ group, or phenyl group (this phenyl group may be substituted with halogen atom, nitro group, cyano group, $C_1$-$C_6$ alkyl group, $C_1$-$C_6$ alkoxy group, $C_1$-$C_6$ haloalkyl group, $C_1$-$C_6$ haloalkoxy group or $NR^8R^9$ group);
$R^8$ and $R^9$ are each independently hydrogen atom or $C_1$-$C_6$ alkyl group;
$R^{12}$ is phenyl group (this phenyl group may be substituted with halogen atom, nitro group, cyano group, $C_1$-$C_6$ alkyl group, $C_1$-$C_6$ alkoxy group, $C_1$-$C_6$ haloalkyl group, $C_1$-$C_6$ haloalkoxy group or $NR^8R^9$ group), or $C_3$-$C_{10}$ heterocyclic group having at least one hetero atom which is selected from oxygen atom, sulfur atom and nitrogen atom and which may be the same or different from each other (this heterocyclic group may be substituted with halogen atom, nitro group, cyano group, $C_1$-$C_6$ alkyl group, $C_1$-$C_6$ alkoxy group, $C_1$-$C_6$ haloalkyl group, $C_1$-$C_6$ haloalkoxy group or $NR^6R^7$ group);
$R^{13}$ is $C_3$-$C_{10}$ heterocyclic group having at least one hetero atom which is selected from oxygen atom, sulfur atom and nitrogen atom and which may be the same or different from each other (this heterocyclic group may be substituted with halogen atom, nitro group, cyano group, $C_1$-$C_6$ alkyl group, $C_1$-$C_6$ alkoxy group, $C_1$-$C_6$ haloalkyl group, $C_1$-$C_6$ haloalkoxy group or $NR^6R^7$ group);
$R^{14}$ is hydrogen atom or $C_1$-$C_6$ alkyl group;
$R^{15}$ and $R^{16}$ are each independently $C_1$-$C_6$ alkyl group or -W-$R^{13}$ group (when either one of $R^{15}$ and $R^{16}$ is -W-$R^{13}$

group, the other may be hydrogen atom);
n is an integer of 0 to 4;
m is an integer of 0 or 1;
Y is oxygen atom or sulfur atom;
$Y^1$ is oxygen atom or sulfur atom; and
W is $C_1$-$C_4$ alkylene group or $C_1$-$C_4$ alkenylene group}.
(5) A plant disease control agent for agricultural or horticultural use, containing, as an active ingredient, a benzisothiazoline derivative or a salt thereof, set forth in (3) or (4).
(6) A pest control agent for agricultural or horticultural use, containing, as an active ingredient, a benzisothiazoline derivative or a salt thereof, set forth in (3) or (4).

Best Mode for Carrying Out the Invention

Explanation is made on the symbols and terms used in the present Description.
"Halogen atom" is fluorine atom, chlorine atom, bromine atom or iodine atom.
The expression such as $C_1$-$C_6$ or the like indicates that the carbon atom(s) of the substituent group which appears after the expression is (are) 1 to 6 in the case of $C_1$-$C_6$.
"$C_1$-$C_6$ alkyl group" indicates a straight chain or branched chain alkyl group having 1 to 6 carbon atoms unless otherwise specified; and there can be mentioned groups such as methyl, ethyl, n-propyl, isopropyl, n-butyl, isobutyl, sec-butyl, tert-butyl, n-pentyl, isopentyl, neopentyl, n-hexyl, 3,3-dimethylbutyl and the like.
"$C_1$-$C_{12}$ alkyl group" indicates a straight chain or branched chain alkyl group having 1 to 12 carbon atoms unless otherwise specified; and there can be mentioned groups such as methyl, ethyl, n-propyl, isopropyl, n-butyl, isobutyl, sec-butyl, tert-butyl, n-nonyl, n-decyl, n-undecyl, n-dodecyl, 3,3-dimethylbutyl and the like.
"$C_3$-$C_{12}$ alkyl group" indicates a straight chain or branched chain alkyl group having 3 to 12 carbon atoms unless otherwise specified; and there can be mentioned groups such as n-propyl, isopropyl, n-butyl, isobutyl, sec-butyl, tert-butyl, n-pentyl, isopentyl, neopentyl, n-nonyl, n-decyl, n-undecyl, n-dodecyl, 3,3-dimethylbutyl and the like.
"$C_3$-$C_6$ cycloalkyl group" indicates a cycloalkyl group having 3 to 6 carbon atoms unless otherwise specified; and there can be mentioned groups such as cyclopropyl, cyclobutyl, cyclopentyl, cyclohexyl and the like.
"$C_1$-$C_6$ haloalkyl group" indicates a straight chain or branched chain alkyl group having 1 to 6 carbon atoms, substituted with halogen atom, unless otherwise specified; and there can be mentioned groups such as fluoromethyl, chloromethyl, difluoromethyl, dichloromethyl, trifluoromethyl, 2,2,2-trifluoroethyl, pentafluoroethyl and the like.
"$C_2$-$C_6$ alkenyl group" indicates a straight chain or branched chain alkenyl group having 2 to 6 carbon atoms unless otherwise specified; and there can be mentioned groups such as vinyl, 1-propenyl, 2-propenyl, isopropenyl, 1-butenyl, 2-butenyl, 3-butenyl and the like.
"$C_2$-$C_6$ haloalkenyl group" indicates a straight chain or branched chain alkenyl group having 2 to 6 carbon atoms, substituted with halogen atom, unless otherwise specified; and there can be mentioned groups such as 2-chlorovinyl, 3-chloro-2-propenyl, 3,3-difluoro-2-methyl-2-propenyl, 3,3-difluoro-2-propenyl, 4,4-difluoro-3-methyl-3-butenyl, 4,4,4-trifluoromethyl-3-methyl-2-butenyl and the like.
"$C_2$-$C_6$ alkynyl group" indicates a straight chain or branched chain alkynyl group having 2 to 6 carbon atoms unless otherwise specified; and there can be mentioned groups such as ethynyl, 1-propynyl, 2-propynyl, 1-butynyl, 2-butynyl, 3-butynyl, 4-methyl-1-pentynyl, 3-methyl-1-pentynyl and the like.
"$C_1$-$C_6$ alkoxy group" indicates an (alkyl)-O- group having 1 to 6 carbon atoms wherein the alkyl portion is defined as above; and there can be mentioned groups such as methoxy, ethoxy, propoxy, isopropoxy, butoxy, pentyloxy, hexyloxy and the like.
"$C_3$-$C_6$ cycloalkyl $C_1$-$C_6$ alkyl group" indicates an alkyl group having 1 to 6 carbon atoms, substituted with a cycloalkyl group having 1 to 6 carbon atoms, wherein the alkyl portion and the cycloalkyl portion are defined as above; and there can be mentioned groups such as cyclopropylmethyl, cyclopentylmethyl, 2-(cyclopropyl)ethyl, 4-(cyclopentyl)butyl, cyclohexylmethyl, cyclohexylethyl and the like.
"$C_1$-$C_6$ alkoxy $C_1$-$C_6$ alkyl group" indicates an alkyl group having 1 to 6 carbon atoms, substituted with an alkoxy group having 1 to 6 carbon atoms, wherein the alkyl portion and the alkoxy portion are defined as above; and there can be mentioned groups such as methoxymethyl, ethoxymethyl, isoproxymethyl, pentyloxymethyl, methoxyethyl, butoxyethyl and the like.
"$C_1$-$C_6$ alkylthio $C_1$-$C_6$ alkyl group" indicates an alkyl group having 1 to 6 carbon atoms, substituted with an (alkyl)-S- group having 1 to 6 carbon atoms, wherein the alkyl portion is defined as above; and there can be mentioned groups such as methylthiomethyl, ethylthiomethyl, isopropylthiomethyl, pentylthiomethyl, methylthioethyl, butylthioethyl and the like.
"$C_1$-$C_6$ haloalkoxy group" indicates a straight chain or branched chain alkyl group having 1 to 6 carbon atoms, substituted with 1 to 13 same or different halogen atoms, wherein the haloalkyl portion is defined as above; and there

can be mentioned groups such as chloromethoxy, difluoromethoxy, chlorodifluoromethoxy, trifluoromethoxy, 2,2,2-trifluoroethoxy and the like.

As "$C_3$-$C_{10}$ heterocyclic group having at least one hetero atom selected from oxygen atom, sulfur atom and nitrogen atom which may be the same or different from each other", there can be mentioned, for example, furan, thiophene, pyrrole, pyrazole, imidazole, pyridine, pyrimidine, pyrazine, pyridazine, pyrrolidine, piperidine, piperazine, morpholine, benzofuran, benzothiophene, indole, benzoxazole, benzothiazole, benzimidazole and the like.

Next, specific examples of the present application compound represented by the general formula [I] are shown in Table 1 to Table 12. However, the present application compound is not restricted to these. Incidentally, some of these compounds are given respective control numbers and appear in the later description.

In the Tables of the present Description, Me indicates methyl group; Et indicates ethyl group; Pr indicates n-propyl group; Pr-i indicates isopropyl group; Bu indicates n-butyl group; Bu-s indicates sec-butyl group; Bu-i indicates isobutyl group; Bu-t indicates tert-butyl group; Pn indicates n-pentyl group; and Pn-c indicates cyclopentyl group. $C_6H_{13}$ indicates n-hexyl group; $C_7H_{15}$ indicates n-heptyl group; Ph indicates phenyl group; and pyridyl indicates pyridyl group. For example, Ph(4-Cl) indicates 4-chlorophenyl group.

Table 1

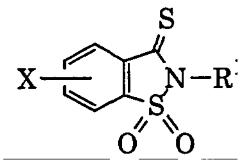

| $R^1$ | X |
|---|---|
| Me | H |
| Et | H |
| Pr | H |
| Pr-i | H |
| Pr-c | H |
| $CH_2CH=CH_2$ | H |
| $CH_2C\equiv CH$ | H |
| Bu | H |
| Bu-i | H |
| Bu-s | H |
| Bu-t | H |
| Pn | H |
| Pn-c | H |
| $C_6H_{13}$ | H |
| $C_7H_{15}$ | H |
| $C_8H_{17}$ | H |
| $C_9H_{19}$ | H |
| $C_{10}H_{21}$ | H |
| $C_{11}H_{23}$ | H |
| $C_{12}H_{25}$ | H |
| Me | 4-OMe |
| Et | 5-Me |
| Pr | 6-Cl |
| Pr-i | 6-$NO_2$ |
| Pr-c | 4-CN |
| $CH_2CH=CH_2$ | 5-$CF_3$ |
| $CH_2C\equiv CH$ | 6-$OCHF_2$ |
| Bu | 4-$NMe_2$ |
| $CHF_2$ | H |
| $CH_2CH_2F$ | H |

Table continued

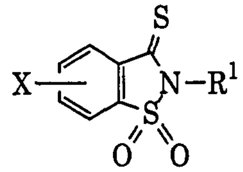

| $R^1$ | X |
|---|---|
| $CH_2CF_3$ | H |
| $CH_2CH_2CF_3$ | H |
| $CH_2CH_2Cl$ | H |
| $CH_2CH_2CH_2Cl$ | H |
| $CH_2CH_2CH_2CH_2CH_2Cl$ | H |

Table 2

| $R^1$ | X |
|---|---|
| $CH_2$Pr-c | H |
| $CH_2$Pn-c | H |
| $CH_2CH_2$Pr-c | H |
| $CH_2CH_2CH_2CH_2$Pn-c | H |
| $CH_2$OMe | H |
| CH(Me)OMe | H |
| $CH_2$OEt | H |
| CH(Me)OEt | H |
| $CH_2$OPr | H |
| $CH_2$OPr-i | H |
| $CH_2$OBu | H |
| $CH_2CH_2$OMe | H |
| $CH_2CH_2$OEt | H |
| $CH_2CH_2$OPr | H |
| $CH_2CH_2$OPr-i | H |
| $CH_2CH_2$OBu | H |
| $CH_2CH_2CH_2$OMe | H |
| $CH_2CH_2CH_2CH_2$OMe | H |
| $CH_2$SMe | H |
| $CH_2$SEt | H |
| $CH_2$SPr | H |
| $CH_2$SBu | H |
| $CH_2CH_2$SMe | H |
| $CH_2CH_2$SEt | H |
| $CH_2CH_2$SPr | H |
| $CH_2CH_2$SPr-i | H |
| $CH_2CH_2$SBu | H |
| $CH_2CH_2CH_2$SMe | H |
| $CH_2CH_2CH_2CH_2$SMe | H |
| $CH_2$COMe | H |
| $CH_2$COEt | H |
| $CH_2$COPr | H |
| $CH_2$COPr-i | H |
| $CH_2$COBu | H |

Table continued

| $R^1$ | X |
|---|---|
| $CH_2COBu$-i | H |
| $CH_2COBu$-t | H |
| $CH_2COPn$ | H |
| $CH_2COCH_2Br$ | H |
| $CH_2COCH_2Cl$ | H |
| $CH_2COCHCl_2$ | H |

Table 3

| $R^1$ | X |
|---|---|
| $CH_2COCF_3$ | H |
| $CH_2CH_2COMe$ | H |
| $CH_2CH_2COEt$ | H |
| $CH_2CH_2CH_2CH_2COMe$ | H |
| $CH_2COPh$ | H |
| $CH_2COPh$(4-F) | H |
| $CH_2COPh$(2-Cl) | H |
| $CH_2COPh$(3-Cl) | H |
| $CH_2COPh$(4-Cl) | H |
| $CH_2COPh$(4-Br) | H |
| $CH_2COPh$(4-OMe) | H |
| $CH_2COPh(4\text{-}NO_2)$ | H |
| $CH_2COPh(4\text{-}OCHF_2)$ | H |
| $CH_2COPh$(4-CN) | H |
| $CH_2COPh(4\text{-}CF_3)$ | H |
| $CH_2COPh(4\text{-}NEt_2)$ | H |
| $CH_2CH_2COPh$ | H |
| $CH_2CH_2COPh$(4-F) | H |
| $CH_2CH_2COPh$(4-Cl) | H |
| $CH_2CH_2CH_2CH_2COPh$ | H |
| $CH_2CONH_2$ | H |
| $CH_2CONHMe$ | H |
| $CH_2CONMe_2$ | H |
| $CH_2CONHCH_2CH=CH_2$ | H |
| $CH_2CONHPh$ | H |
| $CH_2CONHPh$(4-F) | H |
| $CH_2CONHPh$(4-Cl) | H |
| $CH_2CO_2Me$ | H |
| $CH_2CO_2Et$ | H |
| $CH_2CO_2Pr$ | H |
| $CH_2CO_2Pr$-i | H |
| $CH_2CO_2Bu$ | H |
| $CH_2CO_2Pn$ | H |
| $CH_2CO_2C_6H_{13}$ | H |
| $CH_2CO_2CH_2CH=CH_2$ | H |
| $CH_2CO_2CH_2C\equiv CH$ | H |
| $CH_2CO_2Pr$-c | H |
| $CH_2CO_2Pn$-c | H |

Table continued

| $R^1$ | X |
|---|---|
| $CH_2CO_2CH_2CH_2Cl$ | H |
| $CH_2CO_2CH_2CH_2CH_2Cl$ | H |

Table 4

| $R^1$ | X |
|---|---|
| $CH_2CO_2CH_2CH_2CH_2CH_2Br$ | H |
| $CH_2CO_2CH_2CH_2OMe$ | H |
| $CH_2CO_2Ph$ | H |
| $CH_2CO_2Ph(4\text{-}F)$ | H |
| $CH_2CO_2Ph(4\text{-}Cl)$ | H |
| $CH_2CO_2CH_2Ph$ | H |
| $CH_2CO_2CH_2Ph(4\text{-}F)$ | H |
| $CH_2CO_2CH_2Ph(4\text{-}Cl)$ | H |
| $CH(Me)CO_2Me$ | H |
| $CH(Me)CO_2Et$ | H |
| $CH_2OH$ | H |
| $CH_2CH_2OH$ | H |
| $CH_2CN$ | H |
| $CH_2CH_2CN$ | H |
| $CH_2NO_2$ | H |
| $CH_2CH_2NO_2$ | H |
| $CH_2NMe_2$ | H |
| $CH_2CH_2NMe_2$ | H |
| $CH_2CH_2NEtPh$ | H |
| $CH_2Ph$ | H |
| $CH_2Ph(4\text{-}F)$ | H |
| $CH_2Ph(2\text{-}Cl)$ | H |
| $CH_2Ph(3\text{-}Cl)$ | H |
| $CH_2Ph(4\text{-}Cl)$ | H |
| $CH_2Ph(4\text{-}Br)$ | H |
| $CH_2Ph(4\text{-}Me)$ | H |
| $CH_2Ph(4\text{-}OMe)$ | H |
| $CH_2Ph(4\text{-}NO_2)$ | H |
| $CH_2Ph(4\text{-}OCHF_2)$ | H |
| $CH_2Ph(4\text{-}CN)$ | H |
| $CH_2Ph(4\text{-}CF_3)$ | H |
| $CH_2Ph(4\text{-}NMe_2)$ | H |
| Ph | H |
| Ph(4-F) | H |
| Ph(2-Cl) | H |
| Ph(3-Cl) | H |
| Ph(4-Cl) | H |
| Ph(4-Br) | H |
| Ph(4-Me) | H |
| Ph(4-OMe) | H |

Table 5

| $R^1$ | X |
|---|---|
| Ph(4-$NO_2$) | H |
| Ph(4-$OCHF_2$) | H |
| Ph(4-CN) | H |
| Ph(4-$CF_3$) | H |
| Ph(4-$NMe_2$) | H |
| Ph(4-$NPh_2$) | H |
| pyridin-2-yl | H |
| pyridin-3-yl | H |
| pyridin-4-yl | H |
| 2-Cl-pyridin-3-yl | H |
| 5-Cl-pyridin-3-yl | H |
| pyrimidin-2-yl | H |
| furan-2-yl | H |
| 1*H*-pyrrol-2-yl | H |
| thiophen-3-yl | H |
| $CH_2CF_3$ | 4-OMe |
| $CH_2$Pn-c | 5-Me |
| $CH_2$OEt | 6-Cl |
| $CH_2$SMe | 6-$NO_2$ |
| $CH_2$COMe | 4-CN |
| $CH_2CO_2$Me | 5-$CF_3$ |
| $CH_2CO_2$Ph | 6-$OCHF_2$ |
| $CH_2CH_2NO_2$ | 4-$NMe_2$ |
| $CH_2CH_2$CN | 4-OMe |
| $CH_2$Ph | 5-Me |
| Ph | 6-Cl |
| CH=CHCl | H |
| $CH_2CH=CCl_2$ | H |
| $CH_2CH=CF_2$ | H |
| $CH_2CH_2C(Me)=CF_2$ | H |
| $CH_2CH=C(Me)CF_3$ | H |
| COMe | H |
| COEt | H |
| COPr | H |
| COPr-i | H |
| COBu | H |
| COBu-i | H |
| COBu-s | H |
| COBu-t | H |
| COPn | H |

Table 6

| $R^1$ | X |
|---|---|
| $COCH_2Cl$ | H |
| $COCH_2Br$ | H |
| $COCHCl_2$ | H |

Table continued

| $R^1$ | X |
|---|---|
| $COCCl_3$ | H |
| $COCF_3$ | H |
| $COCH_2CH_2Cl$ | H |
| $COCH_2CH_2CH_2CH_2Cl$ | H |
| COPh | H |
| COPh(4-F) | H |
| COPh(4-Cl) | H |
| C(=S)Me | H |
| C(=S)Et | H |
| $C(=S)CF_3$ | H |
| C(=S)Ph | H |
| COOMe | H |
| COOEt | H |
| $COOCH=CH_2$ | H |
| COOPr | H |
| COOPr-i | H |
| COOPr-c | H |
| $COOCH_2CH=CH_2$ | H |
| $COOCH_2C\equiv CH$ | H |
| COOBu | H |
| COOBu-i | H |
| COOBu-s | H |
| COOBu-t | H |
| COOPn | H |
| COOPn-c | H |
| $COOCH_2Cl$ | H |
| $COOCH_2CH_2F$ | H |
| $COOCH_2CH_2Cl$ | H |
| $COOCH_2CH_2CH_2CH_2Cl$ | H |
| $COOCH_2CH_2OMe$ | H |
| COOPh | H |
| COOPh(4-F) | H |
| COOPh(2-Cl) | H |
| COOPh(3-Cl) | H |
| COOPh(4-Cl) | H |
| COOPh(4-Br) | H |
| COOPh(4-Me) | H |

Table 7

| $R^1$ | X |
|---|---|
| COOPh(4-OMe) | H |
| $COOPh(4-NO_2)$ | H |
| $COOPh(4-OCHF_2)$ | H |
| COOPh(4-CN) | H |
| $COOPh(4-CF_3)$ | H |
| $COOCH_2Ph$ | H |
| $COOCH_2Ph(4-F)$ | H |

Table continued

| $R^1$ | X |
|---|---|
| $COOCH_2Ph(2\text{-}Cl)$ | H |
| $COOCH_2Ph(3\text{-}Cl)$ | H |
| $COOCH_2Ph(4\text{-}Cl)$ | H |
| $COOCH_2Ph(4\text{-}Br)$ | H |
| $COOCH_2Ph(4\text{-}Me)$ | H |
| $COOCH_2Ph(4\text{-}OMe)$ | H |
| $COOCH_2Ph(4\text{-}NO_2)$ | H |
| $COOCH_2Ph(4\text{-}OCHF_2)$ | H |
| $COOCH_2Ph(4\text{-}CN)$ | H |
| $COOCH_2Ph(4\text{-}CF_3)$ | H |
| COSMe | H |
| COSEt | H |
| COSPr | H |
| COSPh | H |
| C(=S)OMe | H |
| C(=S)OEt | H |
| C(=S)OPr | H |
| C(=S)OPh | H |
| C(=S)SMe | H |
| C(=S)SEt | H |
| C(=S)SPr | H |
| C(=S)SPh | H |
| COMe | 4-OMe |
| $COCF_3$ | 5-Me |
| COOEt | 6-Cl |
| $COOCH_2CH{=}CH_2$ | $6\text{-}NO_2$ |
| $COOCH_2C{\equiv}CH$ | 4-CN |
| $COOCH_2CH_2Cl$ | $5\text{-}CF_3$ |
| COOPh | $6\text{-}OCHF_2$ |
| $COOCH_2Ph$ | $4\text{-}NMe_2$ |
| COSMe | 4-OMe |
| C(=S)OMe | 5-Me |
| C(=S)SPh | 6-Cl |

Table 8

| $R^1$ | X |
|---|---|
| $CH_2F$ | H |
| $CH_2Cl$ | H |
| $CH_2Br$ | H |
| $CH_2CH{=}CHCl$ | H |
| $CH_2CH{=}CHCOOEt$ | H |
| $CH_2CH{=}CHCOMe$ | H |
| $CH_2$(pyridin-2-yl) | H |
| $CH_2$(pyridin-3-yl) | H |
| $CH_2$(6-Cl-pyridin-3-yl) | H |
| $CH_2$(pyridin-4-yl) | H |
| $CH_2$(2-Cl-pyridin-4-yl) | H |

Table continued

| $R^1$ | X |
|---|---|
| $CH_2$(2,6-$Cl_2$-pyridin-4-yl) | H |
| 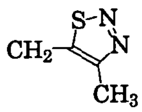 | H |
| $CH_2OC(=O)Ph$ | H |
| $CH_2OC(=O)Ph$(2-Cl) | H |
| $CH_2OC(=O)Ph$(3-Cl) | H |
| $CH_2OC(=O)Ph$(4-Cl) | H |
| $CH_2OC(=O)Ph$(4-Br) | H |
| $CH_2OC(=O)Ph$(4-F) | H |
| $CH_2OC(=O)Ph$(4-OMe) | H |
| $CH_2OC(=O)Ph(4\text{-}NO_2)$ | H |
| $CH_2OC(=O)Ph$(4-CN) | H |
| $CH_2OC(=O)Ph(4\text{-}CF_3)$ | H |
| $CH_2OC(=O)$(pyridin-2-yl) | H |
| $CH_2OC(=O)$(pyridin-3-yl) | H |
| $CH_2OC(=O)$(6-Cl-pyriclin-3-yl) | H |
| $CH_2OC(=O)$(pyridin-4-yl) | H |
| $CH_2OC(=O)$(2-Cl-pyridin-4-yl) | H |
| $CH_2OC(=O)$(2,6-$Cl_2$-pyridin-4-yl) | H |
| 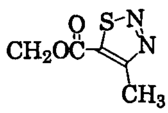 | H |
| $CH_2SCN$ | H |
| $CH_2NCS$ | H |
| $CH_2C(=O)NHCH_2$(pyridin-2-yl) | H |
| $CH_2C(=O)NHCH_2$(pyridin-3-yl) | H |
| $CH_2C(=O)NHCH_2$(6-Cl-pyridin-3-yl) | H |
| $CH_2C(=O)NHCH_2$(pyridin-4-yl) | H |

Table 9

| $R^1$ | X |
|---|---|
| $CH_2C(=O)NHCH_2$(2-Cl-pyridin-4-yl) | H |
| $CH_2C(=O)NHCH_2$(2,6-$Cl_2$-pyridin-4-yl) | H |
| 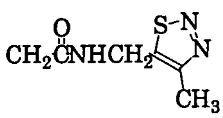 | H |
| $CH_2C(=O)NMeCH_2$(pyridin-2-yl) | H |
| $CH_2C(=O)NMeCH_2$(pyridin-3-yl) | H |
| $CH_2C(=O)NMeCH_2$(6-Cl-pyridin-3-yl) | H |
| $CH_2C(=O)NMeCH_2$(pyridin-4-yl) | H |
| $CH_2C(=O)NMeCH_2$(2-Cl-pyridin-4-yl) | H |
| $CH_2C(=O)NMeCH_2$(2,6-$Cl_2$-pyridin-4-yl) | H |

Table continued

| $R^1$ | X |
|---|---|
| 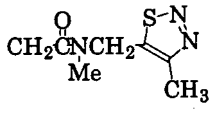 | H |
| $CH(COOEt)_2$ | H |
| CH=CHCOOEt | H |
| CH=CHCOMe | H |
| CH=CHCOPh | H |
| $CH_2C(=S)OEt$ | H |
| $CH(CH_3)CH_2NO_2$ | H |
| $COCH=CH_2$ | H |
| COCH=CHMe | H |
| COCH=CHPh | H |
| COOEt | $4-CH_3$ |
| COOEt | 5-Cl |
| $COO(CH_2)_5CH_3$ | H |
| $COO(CH_2)_7CH_3$ | H |
| $COO(CH_2)_9CH_3$ | H |
| $COOCH_2$(pyridin-2-yl) | H |
| $COOCH_2$(pyridin-3-yl) | H |
| $COOCH_2$(6-Cl-pyridin-3-yl) | H |
| $COOCH_2$(pyridin-4-yl) | H |
| $COOCH_2$(2-Cl-pyridin-4-yl) | H |
| $COOCH_2(2,6-Cl_2$-pyridin-4-yl) | H |
| 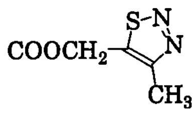 | H |
| $CONHCH_2$(pyridin-2-yl) | H |
| $CONHCH_2$(pyridin-3-yl) | H |
| $CONHCH_2$(6-Cl-pyridin-3-yl) | H |

Table 10

| $R^1$ | X |
|---|---|
| $CONHCH_2$(pyridin-4-yl) | H |
| $CONHCH_2$(2-Cl-pyridin-4-yl) | H |
| $CONHCH_2(2,6-Cl_2$-pyridin-4-yl) | H |
| 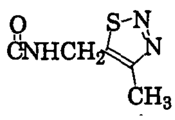 | H |
| $CONMeCH_2$(pyridin-2-yl) | H |
| $CONMeCH_2$(pyridin-3-yl) | H |
| $CONMeCH_2$(6-Cl-pyridin-3-yl) | H |
| $CONMeCH_2$(pyridin-4-yl) | H |
| $CONMeCH_2$(2-Cl-pyridin-4-yl) | H |
| $CONMeCH_2(2,6-Cl_2$-pyridin-4-yl) | H |

Table continued

| $R^1$ | X |
|---|---|
| 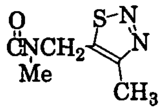 | H |
| $CH_2OCH_2Ph$ | H |
| $CH_2OCH_2Ph(2\text{-}Cl)$ | H |
| $CH_2OCH_2Ph(3\text{-}Cl)$ | H |
| $CH_2OCH_2Ph(4\text{-}Cl)$ | H |
| $CH_2OCH_2Ph(4\text{-}Br)$ | H |
| $CH_2OCH_2Ph(4\text{-}F)$ | H |
| $CH_2OCH_2Ph(4\text{-}OMe)$ | H |
| $CH_2OCH_2Ph(4\text{-}NO_2)$ | H |
| $CH_2OCH_2Ph(4\text{-}CN)$ | H |
| $CH_2OCH_2Ph(4\text{-}CF_3)$ | H |
| $CH_2OCH_2$(pyridin-2-yl) | H |
| $CH_2OCH_2$(pyridin-3-yl) | H |
| $CH_2OCH_2$(6-Cl-pyridin-3-yl) | H |
| $CH_2OCH_2$(pyridin-4-yl) | H |
| $CH_2OCH_2$(2-Cl-pyridin-4-yl) | H |
| $CH_2OCH_2$(2,6-$Cl_2$-pyridin-4-yl) | H |
| 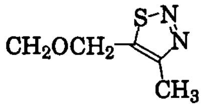 | H |
| $(CH_2)_3OCH_2Ph$ | H |
| $(CH_2)_3OCH_2Ph(2\text{-}Cl)$ | H |
| $(CH_2)_3OCH_2Ph(3\text{-}Cl)$ | H |
| $(CH_2)_3OCH_2Ph(4\text{-}Cl)$ | H |
| $(CH_2)_3OCH_2Ph(4\text{-}Br)$ | H |
| $(CH_2)_3OCH_2Ph(4\text{-}F)$ | H |

Table 11

| $R^1$ | X |
|---|---|
| $(CH_2)_3OCH_2Ph(4\text{-}OMe)$ | H |
| $(CH_2)_3OCH_2Ph(4\text{-}NO_2)$ | H |
| $(CH_2)_3OCH_2Ph(4\text{-}CN)$ | H |
| $(CH_2)_3OCH_2Ph(4\text{-}CF_3)$ | H |
| $(CH_2)_3OCH_2$(pyridin-2-yl) | H |
| $(CH_2)_3OCH_2$(pyridin-3-yl) | H |
| $(CH_2)_3OCH_2$(6-Cl-pyridin-3-yl) | H |
| $(CH_2)_3OCH_2$(pyridin-4-yl) | H |
| $(CH_2)_3OCH_2$(2-Cl-pyridin-4-yl) | H |
| $(CH_2)_3OCH_2$(2,6-$Cl_2$-pyridin-4-yl) | H |
| 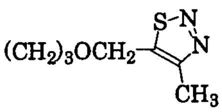 | H |
| $CH_2SCH_2Ph$ | H |

Table continued

| $R^1$ | X |
|---|---|
| $CH_2SCH_2Ph(2\text{-}Cl)$ | H |
| $CH_2SCH_2Ph(3\text{-}Cl)$ | H |
| $CH_2SCH_2Ph(4\text{-}Cl)$ | H |
| $CH_2SCH_2Ph(4\text{-}Br)$ | H |
| $CH_2SCH_2Ph(4\text{-}F)$ | H |
| $CH_2SCH_2Ph(4\text{-}OMe)$ | H |
| $CH_2SCH_2Ph(4\text{-}NO_2)$ | H |
| $CH_2SCH_2Ph(4\text{-}CN)$ | H |
| $CH_2SCH_2Ph(4\text{-}CF_3)$ | H |
| $CH_2SCH_2$(pyridin-2-yl) | H |
| $CH_2SCH_2$(pyridin-3-yl) | H |
| $CH_2SCH_2$(6-Cl-pyridin-3-yl) | H |
| $CH_2SCH_2$(pyridin-4-yl) | H |
| $CH_2SCH_2$(2-Cl-pyridin-4-yl) | H |
| $CH_2SCH_2$(2,6-$Cl_2$-pyridin-4-yl) | H |
| 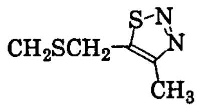 | H |
| $CH_2ON{=}CHPh$ | H |
| $CH_2ON{=}CHPh(2\text{-}Cl)$ | H |
| $CH_2ON{=}CHPh(3\text{-}Cl)$ | H |
| $CH_2ON{=}CHPh(4\text{-}Cl)$ | H |
| $CH_2ON{=}CHPh(4\text{-}Br)$ | H |
| $CH_2ON{=}CHPh(4\text{-}F)$ | H |
| $CH_2ON{=}CHPh(4\text{-}OMe)$ | H |
| $CH_2ON{=}CHPh(4\text{-}NO_2)$ | H |

Table 12

| $R^1$ | X |
|---|---|
| $CH_2ON{=}CHPh(4\text{-}CN)$ | H |
| $CH_2ON{=}CHPh(4\text{-}CF_3)$ | H |
| $CH_2ON{=}CH$(pyridin-2-yl) | H |
| $CH_2ON{=}CH$(pyridin-3-yl) | H |
| $CH_2ON{=}CH$(6-Cl-pyridin-3-yl) | H |
| $CH_2ON{=}CH$(pyridin-4-yl) | H |
| $CH_2ON{=}CH$(2-Cl-pyridin-4-yl) | H |
| $CH_2ON{=}CH$(2,6-$Cl_2$-pyridin-4-yl) | H |
| 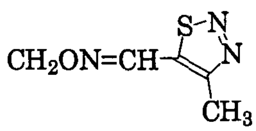 | H |
| $OCH_3$ | H |
| $OCH_2Ph$ | H |
| $OCH_2Ph(2\text{-}Cl)$ | H |
| $OCH_2Ph(3\text{-}Cl)$ | H |
| $OCH_2Ph(4\text{-}Cl)$ | H |

Table continued

| $R^1$ | X |
|---|---|
| $OCH_2Ph(4\text{-}Br)$ | H |
| $OCH_2Ph(4\text{-}F)$ | H |
| $OCH_2Ph(4\text{-}OMe)$ | H |
| $OCH_2Ph(4\text{-}NO_2)$ | H |
| $OCH_2Ph(4\text{-}CN)$ | H |
| $OCH_2Ph(4\text{-}CF_3)$ | H |
| $OCH_2$(pyridin-2-yl) | H |
| $OCH_2$(pyridin-3-yl) | H |
| $OCH_2$(6-Cl-pyridin-3-yl) | H |
| $OCH_2$(pyridin-4-yl) | H |
| $OCH_2$(2-Cl-pyridin-4-yl) | H |
| $OCH_2$(2,6-$Cl_2$-pyridin-4-yl) | H |
| 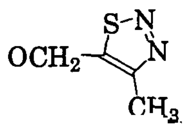 | H |

Representative production processes of the present application compound of the general formula [I] are shown below; however, the production process of the compound is not restricted thereto.

<Production process 1>

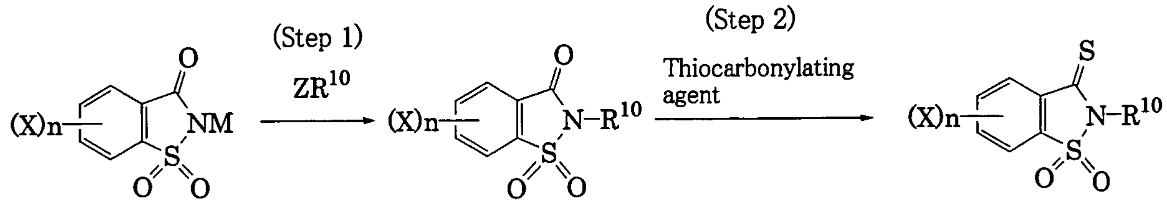


[ III ] [ II ] [ Ia ]

{wherein X and n are defined as above; $R^{10}$ is $C_1$-$C_{12}$ alkyl group, $C_2$-$C_6$ alkenyl group, $C_2$-$C_6$ alkynyl group ,$C_3$-$C_6$ cycloalkyl group, $C_1$-$C_6$ haloalkyl group, $C_2$-$C_6$ haloalkenyl group, $C_3$-$C_6$ cycloalkyl $C_1$-$C_6$ alkyl group, $C_1$-$C_6$ alkoxy $C_1$-$C_6$ alkyl group, $C_1$-$C_6$ alkylthio $C_1$-$C_6$ alkyl group, -C(=Y)-$R^2$ group (wherein Y and $R^2$ are defined as above), -C(=Y)-$Y^1$-$R^3$ group (wherein Y, $Y^1$ and $R^3$ are defined as above), -W-C(=O)-$R^4$ group (wherein W and $R^4$ are defined as above), hydroxy $C_1$-$C_6$ alkyl group, cyano $C_1$-$C_6$ alkyl group, nitro $C_1$-$C_6$ alkyl group, $NR^6R^7$ $C_1$-$C_6$ alkyl group (wherein $R^6$ and $R^7$ are defined as above), or phenyl $C_1$-$C_6$ alkyl group; Z is methanesulfonyloxy group, trifluoromethanesulfonyloxy group, p-toluenesulfonyloxy group or halogen atom; and M is hydrogen atom or sodium atom.}

(Step 1)

A compound represented by the general formula [II] can be produced by reacting a compound [III] with $ZR^{10}$ in a solvent in the presence or absence of a base according to a generally known method [for example, the method described in Journal of the American Chemical Society, Vol. 76, p. 302 (1954) or Vol. 77, p. 5628 (1955), or the method described in JP-A-1974-20779].

The amount of $ZR^{10}$ used in this step may be appropriately selected in a range of 0.5 to 10 mols relative to 1.0 mol of the compound [III] and is preferably 1.0 to 1.2 mols.

As the base usable in the present step, there can be mentioned, for example, metal carbonates such as sodium carbonate, potassium carbonate, magnesium carbonate, calcium carbonate, sodium hydrogencarbonate, potassium hydrogencarbonate and the like; metal acetates such as sodium acetate, potassium acetate, calcium acetate, magnesium

acetate and the like; metal alkoxides such as sodium methoxide, sodium ethoxide, sodium tert-butoxide, potassium methoxide, potassium tert-butoxide and the like; metal hydroxides such as sodium hydroxide, potassium hydroxide, calcium hydroxide, magnesium hydroxide and the like; and metal hydrides such as lithium hydride, sodium hydride, potassium hydride, calcium hydride and the like.

The amount of the base used may be appropriately selected in a range of 0 to 10 mols relative to 1.0 mol of the compound [III] and is preferably 0 to 1.2 mols.

The solvent usable in the present reaction may be any solvent as long as it does not hinder the progress of the present reaction. There can be used, for example, ethers such as diethyl ether, di-isopropyl ether, tetrahydrofuran, dioxane, monoglyme, diglyme and the like; halogenated hydrocarbons such as dichloroethane, chloroform, carbon tetrachloride, tetrachloroethane and the like; aromatic hydrocarbons such as benzene, chlorobenzene, nitrobenzene, toluene, xylene and the like; amides such as N,N-dimethylformamide, N,N-dimethylacetamide and the like; imidazolinones such as 1,3-dimethyl-2-imidazolinone and the like; sulfur compounds such as dimethyl sulfoxide and the like; and nitriles such as acetonitrile and the like. There can also be used mixed solvents thereof.

The reaction temperature may be selected in a range of -20°C to the boiling point of the inert solvent used and preferably is in a range of 0 to 100°C.

The reaction time varies depending upon the reaction temperature, the reaction substrate, the amount of reaction, etc. but is generally 30 minutes to 48 hours.

After the completion of the present reaction, the intended compound [II] is collected from the reaction system according to an ordinary method. The intended compound obtained may be purified as necessary by an operation such as column chromatography, recrystallization or the like.

(Step 2)

A present application compound represented by the general formula [Ia] can be produced by reacting the compound [II] with a thiocarbonylating agent in a solvent or in a solvent-less state according to a generally known method [for example, the method described in Journal of Organic Chemistry, Vol. 16, p. 1582 (1951) or the method described in Indian Journal of Chemistry Section B, Vol. 27, p. 109 (1988)].

The amount of the thiocarbonylating agent used in this step may be selected appropriately in a range of 0.5 to 10 mols relative to 1.0 mol of the compound [II] and is preferably 1.0 to 2.0 mols.

As the thiocarbonylating agent used in the present step, there can be mentioned, for example, phosphorus pentasulfide and Lawesson's reagent [2,4-bis(4-methoxyphenyl)-1,3-dithia-2,4-diphosphetane-2,4-disulfide].

The solvent usable in the present reaction may be any solvent as long as it does not hinder the progress of the present reaction. There can be used, for example, ethers such as diethyl ether, di-isopropyl ether, tetrahydrofuran, dioxane, monoglyme, diglyme and the like; halogenated hydrocarbons such as dichloroethane, chloroform, carbon tetrachloride, tetrachloroethane and the like; aromatic hydrocarbons such as benzene, chlorobenzene, nitrobenzene, toluene, xylene and the like; amides such as N,N-dimethylformamide, N,N-dimethylacetamide and the like; imidazolinones such as 1,3-dimethyl-2-imidazolinone and the like; sulfur compounds such as dimethyl sulfoxide and the like; nitriles such as acetonitrile and the like; and organic base compounds such as pyridine and the like. There can also be used mixed solvents thereof.

The reaction temperature may be selected in a range of -20°C to the boiling point of the inert solvent used and preferably is in a range of 0 to 150°C.

The reaction time varies depending upon the reaction temperature, the reaction substrate, the amount of reaction, etc. but is generally 30 minutes to 48 hours.

After the completion of the present reaction, the intended compound [Ia] is collected from the reaction system according to an ordinary method. The intended compound obtained may be purified as necessary by an operation such as column chromatography, recrystallization or the like.

<Production process 2>

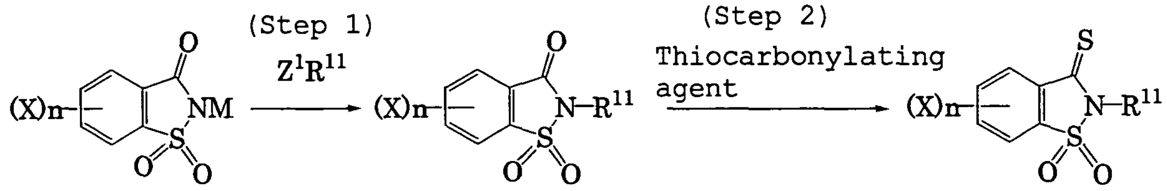


[III] [IIa] [Ib]

{wherein X, n and M are as defined above; $R^{11}$ is phenyl group [this phenyl group may be substituted with halogen atom, nitro group, cyano group, $C_1$-$C_6$ alkyl group, $C_1$-$C_6$ alkoxy group, $C_1$-$C_6$ haloalkyl group, $C_1$-$C_6$ haloalkoxy group or $NR^6R^7$ group (wherein $R^6$ and $R^7$ are as defined above)], or $C_3$-$C_{10}$ heterocyclic group having at least one hetero atom which is selected from oxygen atom, sulfur atom and nitrogen atom and which may be the same or different from each other [this heterocyclic group may be substituted with halogen atom, nitro group, cyano group, $C_1$-$C_6$ alkyl group, $C_1$-$C_6$ alkoxy group, $C_1$-$C_6$ haloalkyl group, $C_1$-$C_6$ haloalkoxy group or $NR^6R^7$ group (wherein $R^6$ and $R^7$ are as defined above)]; and $Z^1$ is $B(OH)_2$, $Pb(OC(=O)Me)_3$, trifluoromethanesulfonyloxy group or halogen atom.}

(Step 1)

A compound represented by the general formula [IIa] can be produced by reacting a compound [III] with $Z^1R^{11}$ in a solvent in the presence of a metal catalyst in the presence or absence of a base according to a generally known method [for example, the method described in Journal of Organic Chemistry, Vol. 61, No. 17, p. 5865 (1996) or the method described in Tetrahedron Letter, Vol. 39, No. 19, p. 2933 (1998)].

The amount of $Z^1R^{11}$ used in this step may be appropriately selected in a range of 0.5 to 10 mols relative to 1 mol of the compound [III] and is preferably 1.0 to 3.0 mols.

As the base usable in the present step, there can be mentioned, for example, metal carbonates such as sodium carbonate, potassium carbonate, magnesium carbonate, calcium carbonate, sodium hydrogencarbonate, potassium hydrogencarbonate and the like; metal acetates such as sodium acetate, potassium acetate, calcium acetate, magnesium acetate and the like; metal alkoxides such as sodium methoxide, sodium ethoxide, sodium tert-butoxide, potassium methoxide, potassium tert-butoxide and the like; metal hydroxides such as sodium hydroxide, potassium hydroxide, calcium hydroxide, magnesium hydroxide and the like; metal hydrides such as lithium hydride, sodium hydride, potassium hydride, calcium hydride and the like; and organic bases such as pyridine, triethylamine, 1,8-diazabicyclo[5.4.0]-7-undecene and the like.

The amount of the base used in the present step may be appropriately selected in a range of 0 to 10 mols relative to 1.0 mol of the compound [III] and is preferably 0 to 3.0 mols.

As the metal catalyst usable in the present step, there can be mentioned, for example, copper (II) acetate, copper (I) iodide, tetrakis(triphenylphosphine) palladium (0), palladium (II) acetate, bis(dibenzylideneacetone) palladium (0), bis(triphenylphosphine) palladium (II) dichloride, bis(tri-o-tolylphosphine) palladium (II) dichloride, bis(triphenylphosphine) palladium (II) diacetate, and bis(triphenylphosphine) nickel (II) dichloride.

The amount of the metal catalyst used in the present step is selected appropriately in a range of 0.1 to 10 mols relative to 1 mol of the compound [III] and is preferably 0.1 to 2.0 mols.

The solvent usable in the present reaction may be any solvent as long as it does not hinder the progress of the present reaction. There can be used, for example, ethers such as diethyl ether, di-isopropyl ether, tetrahydrofuran, dioxane, monoglyme, diglyme and the like; halogenated hydrocarbons such as dichloroethane, chloroform, carbon tetrachloride, tetrachloroethane and the like; aromatic hydrocarbons such as benzene, chlorobenzene, nitrobenzene, toluene, xylene and the like; amides such as N,N-dimethylformamide, N,N-dimethylacetamide and the like; imidazolinones such as 1,3-dimethyl-2-imidazolinone and the like; sulfur compounds such as dimethyl sulfoxide and the like; nitriles such as acetonitrile and the like; and organic base compounds such as pyridine, triethylamine and the like. There can also be used mixed solvents thereof.

The reaction temperature may be selected in a range of -20°C to the boiling point of the inert solvent used and preferably is in a range of 0 to 150°C.

The reaction time varies depending upon the reaction temperature, the reaction substrate, the amount of reaction, etc. but is generally 30 minutes to 48 hours.

After the completion of the present reaction, the intended compound [IIa] is collected from the reaction system according to an ordinary method. The intended compound obtained may be purified as necessary by an operation such as column chromatography, recrystallization or the like.

(Step 2)

A present application compound represented by the general formula [Ib] can be produced based on the step 2 of the production process 1.

<Production process 3>

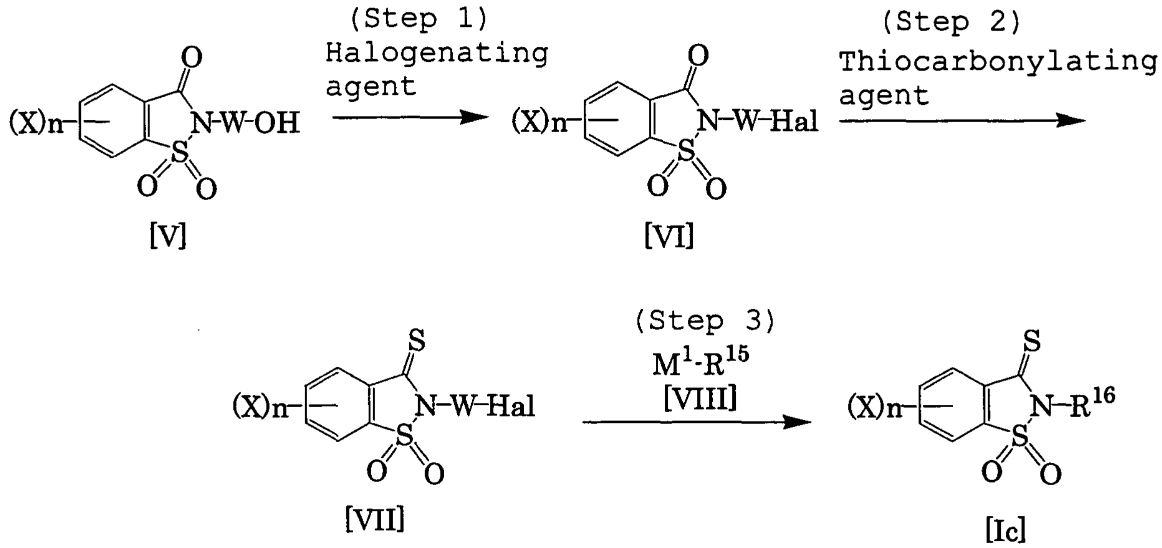


[V] [VI] [VII] [Ic]

{wherein Hal is halogen atom; $M^1$ is hydrogen atom or alkali metal atom; $R^{15}$ is $C_1$-$C_6$ alkoxy group, $C_1$-$C_6$ alkylthio group, $-Y^1$-W-$R^{12}$ group, $-(Y^1)_m$-(C(=Y))$_m$-$R^{12}$ group, $-Y^1$-N=C$R^{14}R^{12}$ group, cyano group, cyanothio group, isothiocyano group or nitro group; $R^{16}$ is $C_1$-$C_6$ alkoxy $C_1$-$C_6$ alkyl group, $C_1$-$C_6$ alkylthio $C_1$-$C_6$ alkyl group, -W-$Y^1$-W-$R^{12}$ group, -W-$(Y^1)_m$-(C(=Y))$_m$-$R^{12}$ group, -W-$Y^1$-N=C$R^{14}R^{12}$ group, cyano $C_1$-$C_6$ alkyl group, cyanothio $C_1$-$C_6$ alkyl group, isothiocyano $C_1$-$C_6$ alkyl group, or nitro $C_1$-$C_6$ alkyl group; and X, Y, $Y^1$, $R^{12}$, $R^{14}$, m, n and W are as defined above.}

A compound represented by the general formula [V] can be produced according to a generally known method [for example, the method described in Journal of Heterocyclic Chemistry, Vol. 26, p. 1073 (1989)].

(Step 1)

A compound represented by the general formula [VI] can be produced by reacting the compound [V] with a halogenating agent in the presence or absence of a solvent according to a generally known method [for example, the method described in Journal of Medicinal Chemistry, Vol. 36, p. 3178 (1993) or the method described in Journal of Medicinal Chemistry, Vol. 37, p. 2623 (1994)].

The amount of the halogenating agent used in the present step may be selected appropriately in a range of 0.5 to 10 mols relative to 1.0 mol of the compound [V] and is preferably 1.0 to 2.0 mols.

As the halogenating agent usable in the present step, there can be mentioned, for example, hydrogen chloride, thionyl chloride, phosphorus pentachloride, hydrogen bromide and phosphorus tribromide.

The solvent usable in the present reaction may be any solvent as long as it does not hinder the progress of the present reaction. There can be used, for example, ethers such as diethyl ether, diisopropyl ether, tetrahydrofuran, dioxane, monoglyme, diglyme and the like; halogenated hydrocarbons such as dichloroethane, chloroform, carbon tetrachloride, tetrachloroethane and the like; aromatic hydrocarbons such as benzene, chlorobenzene, nitrobenzene, toluene, xylene and the like; acids such as acetic acid and the like; and water. There can also be used mixed solvents thereof.

The reaction temperature may be selected in a range of -20°C to the boiling point of the inert solvent used and preferably is in a range of 0 to 100°C.

The reaction time varies depending upon the reaction temperature, the reaction substrate, the amount of reaction, etc. but is generally 30 minutes to 24 hours.

After the completion of the present reaction, the intended compound [VI] is collected from the reaction system according to an ordinary method. The intended compound obtained may be purified as necessary by an operation such as column chromatography, recrystallization or the like.

(Step 2)

A present application compound represented by the general formula [VII] can be produced based on the step 2 of the production method 1.

(Step 3)

A compound represented by the general formula [Ic] can be produced by reacting the compound [VII] with a compound [VIII] in a solvent in the presence or absence of a base.

The amount of the compound [VIII] used in the present step is selected appropriately in a range of 0.5 to 10 mols relative to 1.0 mol of the compound [VII] and is preferably 1.0 to 1.2 mols.

As the base usable in the present step, there can be mentioned, for example, metal carbonates such as sodium carbonate, potassium carbonate, magnesium carbonate, calcium carbonate, sodium hydrogencarbonate, potassium hydrogencarbonate and the like; metal acetates such as sodium acetate, potassium acetate, calcium acetate, magnesium acetate and the like; metal alkoxides such as sodium methoxide, sodium ethoxide, sodium tert-butoxide, potassium methoxide, potassium tert-butoxide and the like; metal hydroxides such as sodium hydroxide, potassium hydroxide, calcium hydroxide, magnesium hydroxide and the like; metal hydrides such as lithium hydride, sodium hydride, potassium hydride, calcium hydride and the like; and organic bases such as triethylamine, pyridine and the like.

The amount of the base used may be appropriately selected in a range of 0 to 10 mols relative to 1 mol of the compound [VII] and is preferably 0 to 1.2 mols.

The solvent usable in the present reaction may be any solvent as long as it does not hinder the progress of the present reaction. There can be used, for example, ethers such as diethyl ether, di-isopropyl ether, tetrahydrofuran, dioxane, monoglyme, diglyme and the like; halogenated hydrocarbons such as dichloroethane, chloroform, carbon tetrachloride, tetrachloroethane and the like; aromatic hydrocarbons such as benzene, chlorobenzene, nitrobenzene, toluene, xylene and the like; amides such as N,N-dimethylformamide, N,N-dimethylacetamide and the like; imidazolinones such as 1,3-dimethyl-2-imidazolinone and the like; sulfur compounds such as dimethyl sulfoxide and the like; nitriles such as acetonitrile and the like; acids such as acetic acid and the like; and water. There can also be used mixed solvents thereof.

The reaction temperature may be selected in a range of -20°C to the boiling point of the inert solvent used and preferably is in a range of 0 to 100°C.

The reaction time varies depending upon the reaction temperature, the reaction substrate, the amount of reaction, etc. but is generally 30 minutes to 24 hours.

After the completion of the present reaction, the intended compound [Ic] is collected from the reaction system according to an ordinary method. The intended compound obtained may be purified as necessary by an operation such as column chromatography, recrystallization or the like.

<Production process 4>

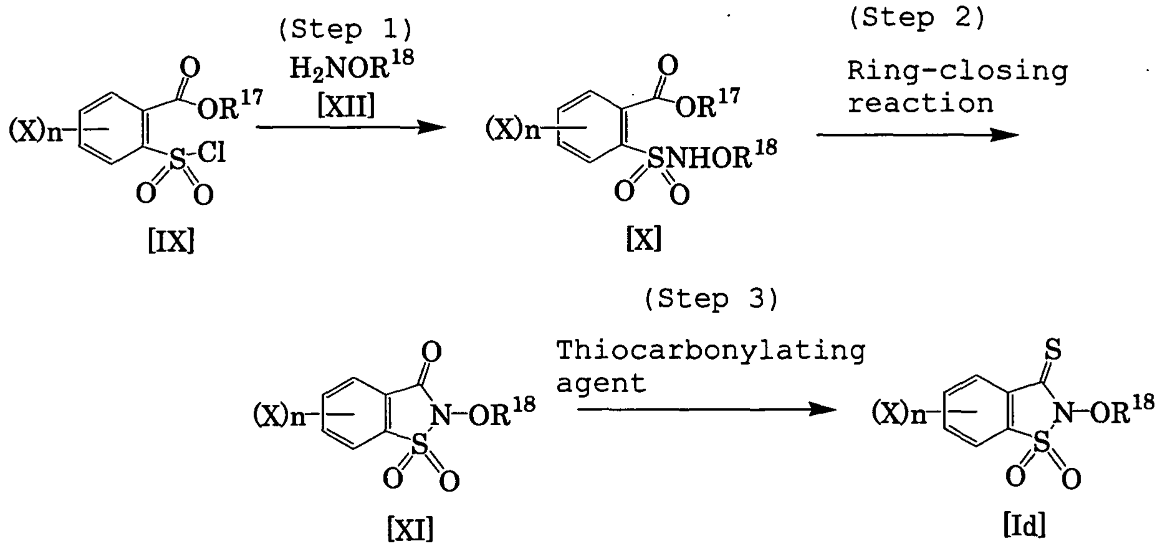


[IX] [X] [XI] [Id]

(wherein $R^{17}$ is $C_1$-$C_4$ alkyl; $R^{18}$ is $C_1$-$C_{12}$ alkyl group, $C_2$-$C_6$ alkenyl group, $C_2$-$C_6$ alkynyl group, $C_3$-$C_6$ cycloalkyl group, $C_1$-$C_6$ haloalkyl group, $C_2$-$C_6$ haloalkenyl group, $C_3$-$C_6$ cycloalkyl $C_1$-$C_6$ alkyl group, or -W-$R^{12}$ group; and $R^{12}$, X, n and W are as defined above.)

(Step 1)

A compound represented by the general formula [X] can be produced by reacting a compound [IX] with a compound [XII] or a salt thereof in the presence of a base in the presence or absence of a solvent according to a generally known method [for example, the method described in Journal of Medicinal Chemistry, Vol. 38, p. 1865 (1995)].
The amount of the compound [XII] used in this step may be appropriately selected in a range of 0.5 to 10 mols relative to 1.0 mol of the compound [IX] and is preferably 1.0 to 1.2 mols.
As the base usable in the present step, there can be mentioned, for example, metal carbonates such as sodium carbonate, potassium carbonate, magnesium carbonate, calcium carbonate, sodium hydrogencarbonate, potassium hydrogencarbonate and the like; metal acetates such as sodium acetate, potassium acetate, calcium acetate, magnesium acetate and the like; metal alkoxides such as sodium methoxide, sodium ethoxide, sodium tert-butoxide, potassium methoxide, potassium tert-butoxide and the like; metal hydroxides such as sodium hydroxide, potassium hydroxide, calcium hydroxide, magnesium hydroxide and the like; metal hydrides such as lithium hydride, sodium hydride, potassium hydride, calcium hydride and the like; and organic bases such as triethylamine, pyridine and the like.
The amount of the base used may be appropriately selected in a range of 0 to 10 mols relative to 1 mol of the compound [IX] and is preferably 0 to 1.2 mols.
The solvent usable in the present reaction may be any solvent as long as it does not hinder the progress of the present reaction. There can be used, for example, ethers such as diethyl ether, di-isopropyl ether, tetrahydrofuran, dioxane, monoglyme, diglyme and the like; halogenated hydrocarbons such as dichloroethane, chloroform, carbon tetrachloride, tetrachloroethane and the like; aromatic hydrocarbons such as benzene, chlorobenzene, nitrobenzene, toluene, xylene and the like; amides such as N,N-dimethylformamide, N,N-dimethylacetamide and the like; imidazolinones such as 1,3-dimethyl-2-imidazolinone and the like; sulfur compounds such as dimethyl sulfoxide and the like; nitriles such as acetonitrile and the like; and water. There can also be used mixed solvents thereof.
The reaction temperature may be selected in a range of -20°C to the boiling point of the inert solvent used and preferably is in a range of 0 to 30°C.
The reaction time varies depending upon the reaction temperature, the reaction substrate, the amount of reaction, etc. but is generally 30 minutes to 24 hours.

(Step 2)

A compound represented by the general formula [XI] can be produced by heating a compound [X] in the presence or absence of an acid in the presence or absence of a solvent according to a generally known method [for example, the method described in Journal of Medicinal Chemistry, Vol. 38, p. 1865 (1995)].
As the acid usable in the present reaction, there can be mentioned, for example, mineral acids such as polyphosphoric acid, hydrochloric acid, sulfuric acid and the like; and organic acids such as acetic acid, methanesulfonic acid, p-toluenesulfonic acid and the like.
The solvent usable in the present reaction may be any solvent as long as it does not hinder the progress of the present reaction. There can be used, for example, ethers such as tetrahydrofuran, dioxane, monoglyme, diglyme and the like; and aromatic hydrocarbons such as benzene, chlorobenzene, nitrobenzene, toluene, xylene and the like. There can also be used mixed solvents thereof.
The reaction temperature may be selected in a range of 70°C to the boiling point of the inert solvent used and preferably is in a range of 100 to 200°C.
The reaction time varies depending upon the reaction temperature, the reaction substrate, the amount of reaction, etc. but is generally 1 to 72 hours.
After the completion of the present reaction, the intended compound [XI] is collected from the reaction system according to an ordinary method. The intended compound obtained may be purified as necessary by an operation such as column chromatography, recrystallization or the like.

(Step 3)

A present application compound represented by the general formula [Id] can be produced based on the step 2 of the production process 1.
In using the present application compound represented by the general formula [I] as a plant disease control agent for agricultural or horticultural use or as a pest control agent for agricultural or horticultural use, an active ingredient is selected and can be used in an appropriate formulation depending upon the intended application. Ordinarily, the active ingredient is diluted with an inert liquid or solid carrier, is mixed with a surfactant, etc. as necessary, and can be used in a control agent formulation such as dust, wettable powder, emulsifiable concentrate, granule or the like.
The proportion of the active ingredient used is determined appropriately as required. An appropriate proportion

is 0.1 to 50% by weight in the case of dust or granule, and 5 to 80% by weight in the case of emulsifiable concentrate or wettable powder.

As the carrier used in formulating the active ingredient into a control agent formulation, there can be mentioned, for example, solid carriers such as talc, bentonite, clay, kaolin, diatomaceous earth, white carbon, vermiculite, calcium carbonate, slaked lime, siliceous sand, ammonium sulfate, urea and the like; and liquid carriers such as isopropyl alcohol, xylene, cyclohexane, methylnaphthalene and the like.

As the surfactant or dispersing agent, there can be mentioned, for example, metal alkylbenzenesulfonate, metal dinaphthylmethanedisulfonate, alcohol sulfate salt, alkylarylsulfonic acid salt, lignosulfonic acid salt, polyoxyethylene glycol ether, polyoxyethylene alkyl aryl ether, polyoxyethylene sorbitan monoalkylate and the like. As the auxiliary agent, there can be mentioned, for example, carboxymethyl cellulose, polyethylene glycol and gum arabic. The control agent obtained is applied after dilution into an appropriate concentration, or is applied directly.

In actual use of the control agent obtained, the agent can be used per se, or can be used after dilution with a diluent (e.g. water) into a desired concentration. The application of the control agent containing the present application compound or of the dilution product thereof can be conducted by an ordinary application method such as spreading (e.g. spraying, misting, atomizing, powder spreading, granule spreading, submerged application, or application to seedling-raising box), application to soil (e.g. mixing or drenching), application to surface (e.g. painting, dressing or covering), dipping, poisonous feed, application by fumigation, or the like. It is also possible to raise domestic animals with a feed containing the above active ingredient in order to prevent the emergence or growth of harmful pests, particularly harmful insects present in their excrements. The control agent may also be applied by small amount spreading in ultra-high concentration. In this application, the active ingredient may be used in a 100 % concentration. The proportion of the active ingredient used in the control agent is determined appropriately as required, and an appropriate proportion is 0.1 to 20% by weight in the case of dust or granule and 1 to 80% by weight in the case of emulsifiable concentrate or wettable powder. The application amount of the control agent varies depending upon the kind of the compound used, the plant disease targeted, the pest targeted, the tendency of emergence, the extent of damage, the conditions of environment, the formulation of the control agent used, etc.

For example, in the case of the powder or granule which is used per se, the amount of the active ingredient is selected appropriately in a range of 0.1 g to 5 kg, preferably 1 g to 1 kg per 10 ares. In the case of the emulsifiable concentrate or wettable powder which is used in a liquid state, the active ingredient is selected appropriately in a range of 0.1 to 10,000 ppm, preferably 10 to 3,000 ppm. When the control agent is applied into a seedling-raising box, it can be prepared in a formulation in which the release of active ingredient has been controlled, in order to allow the formulation to have a long-term activity.

By being applied in the above-mentioned formulation, the present application compound represented by the general formula [I] can control plant diseases caused by molds, bacteria or virus.

Non-limiting specific examples of the plant diseases, there can be mentioned, for example, Pseudoperonospora cubensis, Venturia inaequalis, Erysiphe graminis, Septoria nodorum, Pyricularia oryzae, Botrytis cinerea, Rhizoctonia solani, Puccinia recondita, Xanthomonas oryzae, Burkholderia glumae, Burkholderia plantarii, Fusarium spp., Pythium spp., Rhizopus spp., Trichoderma viride, Rhizoctonia solani, Acidovorax avenae, Fusarium moniliforme and Erwinia ananas.

The present application compound shows excellent control effects to Hemiptera pests, Lepidoptera pests, Coleoptera pests, Diptera pests, Hymenoptera pests, Orthoptera pests, Isoptera pests, Thysanoptera pests, spider mites, plant-parasitic nematodes, etc. Such pests can be exemplified below.

Examples of Hemiptera pests are as follows. Heteroptera such as Riptortus clavatus, Nezara viridula, Lygus sp., Blissus leucopterus, Stephanitis nashi and the like; leafhoppers such as nephotettix cincticeps, Empoasca sp., Erythroneura sp., Circulifer sp. and the like; delphacid planthoppers such as Nilaparvata lugens, Sogatella furcifera, Laodelphax striatellus and the like; jumping plantlice such as Psylla sp. and the like; whiteflies such as Bemisia tabaci, Trialeurodes vaporariorum and the like; aphides such as Viteus vitifolii, Myzus persicae, Aphis pomi, Aphis gossypii, Aphis fabae, Rhopalosiphum pseudobrassicas, Aulacorthum solani, Schizaphis graminum and the like; mealy bugs such as Pseudococcus comstocki, Ceroplastes rubens, Comstockaspis perniciosa, Unaspis yanomensis and the like; and Rhodnius sp.

Examples of Lepidoptera pests are as follows. Leafroller moths such as Homona magnanima, Adoxophyes orana, Sparganothis pilleriana, Grapholitha molesta, Leguminivora glycinivorella, laspeyresia pomonella, Eucosma sp., Lobesia botrana and the like; cochylid moths such as Eupoecillia ambiguella and the like; bagworm moths such as Bambalina sp. and the like; tineids such as Nemapogon granellus, Tinea translucens and the like; lyonetid moths such as Lyonetia prunifoliella and the like; leafblotch miners such as Phyllonorycter rigoniella and the like; phyllocnistidae such as Phyllocnistis citrella and the like; yponomeutids such as Plutella xylostella, Prays citri and the like; clearwing moths such as Paranthrene regalis, Synanthedon sp. and the like; gelechiid moths such as Pectinophora gossypiela, Phthorimaea operculella, Stomopteryx sp. and the like; fruitworm moths such as Carposina niponensis and the like; slug caterpillar moths such as Monema flavescens and the like; pyralid moths such as Chilo suppressalis, Cnaphalocrocis

medinalis, Ostrinia nubilalis, Ostrinia furnacalis, Hellula undalis, Galleria mellonella, Elasmopalpus lignosellus, Loxostege sticticalis and the like; whites such as Pieris rapae and the like; geometrid moths such as Ascotis selenaria and the like; tent caterpillar moths such as Malacosoma nuestria and the like; sphinx moths such as Manduca sexta and the like; tussock moths such as Euproctis pseudoconspersa, Lymantria dispar and the like; tiger moths such as Hyphantria cunea and the like; and owlet moths such as Heliothis virescens, Helicoverpa zea, Spodoptera exigua, Helicoverpa armigera, Spodoptera litura, Mamestra brassicae, Agrotis ipsiron, Pseudaletia separata, Trichoplusia ni and the like.

Examples of Coleoptera pests are as follows. Chafers such as Anomala cuprea, Popillia japonica, Anomala rufocuprea, Eutheola rugiceps and the like; click beetles such as Agriotes sp., Conodeus sp. and the like; ladybirds such as Epilachna vigintioctopunctata, Epilachna varivestis and the like; darkling beetles such as Tribolium castaneum and the like; longhorned beetles such as Anoplophora malasiaca, Monochamus alternatus and the like; seed beetles such as Acanthoscelides obtectus, Callosobruchus chinensis and the like; leaf beetles such as Leptinotarsa decemlineata, Diabrotica sp., Oulema oryzae, Chaetocnema concinna, Phaedon cochlearias, Oulema melanopus, Dicladispa armigera and the like; apionidae such as Apion godnami and the like; weevils such as Lissorhoptrus oryzophilus, Anthonomus grandis and the like; rhynchophoridae such as Sitophilus zeamais and the like; bark beetles; dermestid beetles; and drugstore beetles.

Examples of Diptera pests are as follows. Tipra ano, Tanytarsus oryzae, Orseolia oryzae, Ceratitis capitata, Hydrellia griseola, Drosophila suzukii, Oscinella frit, Chlorops oryzae, Ophiomyia phaseoli, Liriomyza trifolii, Pegomya hyoscyami, Hylemia platura, Atherigona soccata, Musca domestica, Gastrophilus sp., Stomoxys sp., Aedes aegypti, Culex pipiens, Anopheles slnensis and Culex tritaeniorhynchus.

Examples of Hymenoptera pests are as follows. Cephus sp., Harmolita sp., Athalia sp., Vespa sp. and fire ants.

Examples of Orthoptera pests are as follows. Blatella germanica, Periplaneta Americana, Gryllotalpa africana, Locusta migratoria migratoriodes, and Melanoplus sanguinipes.

Examples of Isoptera pests are as follows. Reticulitermes speratus and Coptotermes formosanus.

Examples of Thysanoptera pests are as follows. Scirtothrips dorsalis, Thrips palmi, Heliothrips haemorrhoidalis, Frankliniella occidentalis and Haplothrips aculeatus.

Examples of spider mites are as follows. Tetranychus urticae, Tetranychus kanzawai, Panonychus citri, Panonychus ulmi, Eotetranychus carpini, Eotetranychus banksi, Phyllocoptruta oleivora, Polyphagotarsonemus latus, Brevipalpus sp., Rhizoglyphus robini and Tyrophagus putrescentiae.

Examples of plant-parasitic nematodes are as follows. Meloidogyne incognita, Pratylenchus sp., Heterodera glycines, Aphelenchoides besseyi and Bursaphelenchus xylophilus.

Besides, there can also be mentioned harmful animals, offensive animals, public health pests, parasites, such as Gastroposa (e.g. Pomacea canaliculata, Incilaria sp. and Achatina fulica), Isopoda (e.g. Armadillidium sp., sow bug and centipede), booklice (e.g. Liposcelis sp.), silverfish (e.g. Ctenolepisma sp.), fleas (e.g. Pulex sp. and Ctenocephalides sp.), chewing lice (e.g. Trichodectes sp.), bed bugs (e.g. Cimex sp.), animal-parasitic mites (e.g. Boophilus microplus and Haemaphysalis longicornis), epidermoptidae, and the like.

The present application compound represented by the general formula [I] may be mixed as necessary with known insecticides, plant disease control agents, herbicides, plant growth-regulating agents, fertilizers, etc.

Examples

In the following Examples, description is made in detail on the production processes of present application compound derivatives represented by the general formula [I] used in the plant disease control agent or pest control agent for agricultural or horticultural use according to the present invention and intermediates for synthesis of such derivatives, as well as on the production method and application of such control agent. However, the present invention is in no way restricted to these Examples.

<Example 1>

Production of 2-ethoxymethyl-1,2-benzisothiazoline-3-thioxo-1,1-dioxide (compound No. 3)

(1) Production of 2-ethoxymethyl-1,2-benzisothiazoline-3-oxo-1,1-dioxide

1.0 g (4.9 mmole) of sodium saccharin anhydrous was dissolved in 20 ml of N,N-dimethylformamide. Thereto was added 0.56 g (5.9 mmole) of chloromethyl ethyl ether. The mixture was stirred at room temperature for 2 hours to give rise to a reaction. After confirmation of the completion of the reaction, the reaction mixture was poured into water, followed by extraction with ethyl acetate. The resulting organic layer was washed with water and dried over anhydrous magnesium sulfate. The inorganic matter was removed by filtration and the filtrate was subjected to distillation under reduced pressure to remove the solvent. The residue was washed with isopropyl ether to obtain 1.0 g (yield: 85%) of 2-ethoxyethyl-1,2-benzisothiazoline-3-oxo-1,1-dioxide as a white powder (melting point: 82 to 84°C).

$^1$H-NMR data ($CDCl_3$/TMS δ (ppm)): 1.26 (3H, t), 3.73 (2H, q), 5.29 (2H, s), 7.9 (3H. m), 8.1 (1H, d)

(2) Production of 2-ethoxymethyl-1,2-benzisothiazoline-3-thioxo-1,1-dioxide (compound No. 3)

0.5 g (2.1 mmole) of 2-ethoxymethyl-1,2-benzisothiazoline-3-oxo-1,1-dioxide was dissolved in 20 ml of xylene. Thereto was added 0.46 g (2.1 mmole) of phosphorus pentasulfide. The mixture was stirred for 1 hour under refluxing with heating, to give rise to a reaction. After confirmation of the completion of the reaction, the reaction mixture was cooled to room temperature. The inorganic matter was removed by filtration. The filtrate was subjected to distillation under reduced pressure to remove the solvent. The residue was purified by silica gel column chromatography to obtain 0.1 g (yield: 19%) of 2-ethoxymethyl-1,2-benzisothiazoline-3-thioxo-1,1-dioxide as a yellow powder (melting point: 76 to 78°C).

$^1$H-NMR data ($CDCl_3$/TMS δ (ppm)): 1.26 (3H, t), 3.78 (2H, q), 5.61 (2H, s), 7.85 (3H, m), 8.29 (1H, d)

<Example 2>

Production of 2-(2,2,2-trifluoroethyl)-1,2-benzisothiazoline-3-thioxo-1,1-dioxide (compound No. 1)

(1) Production of 2-(2,2,2-trifluoroethyl)-1,2-benzisothiazoline-3-oxo-1,1-dioxide

5.0 g (24.4 mmole) of anhydrous sodium saccharinate was dissolved in 50 ml of N,N-dimethylformamide. Thereto was added 6.7 g (28.9 mmole) of 2,2,2-trifluoroethyl trifluoromethanesulfonate. The mixture was stirred at room temperature for 3 days to give rise to a reaction. After confirmation of the completion of the reaction, the reaction mixture was poured into water, followed by extraction with ethyl acetate. The resulting organic layer was washed with water and dried over anhydrous magnesium sulfate. The inorganic matter was removed by filtration. The filtrate was subjected to distillation under reduced pressure to remove the solvent, whereby was obtained 5.13 g of 2-(2,2,2-trifluoroethyl)-1,2-benzisothiazoline-3-oxo-1,1-dioxide as a white powder.

(2) Production of 2-(2,2,2-trifluoroethyl)-1,2-benzisothiazoline-3-thioxo-1,1-dioxide (compound No. 1)

0.5 g (1.9 mmole) of 2-(2,2,2-trifluoroethyl)-1,2-benzisothiazoline-3-oxo-1,1-dioxide was dissolved in 20 ml of xylene. Thereto was added 0.42 g (1.9 mmole) of phosphorus pentasulfide. The mixture was stirred for 6 hours under refluxing with heating, to give rise to a reaction. After confirmation of the completion of the reaction, the reaction mixture was cooled to room temperature. The inorganic matter was removed by filtration. The filtrate was subjected to distillation under reduced pressure to remove the solvent. The residue was purified by silica gel column chromatography to obtain 0.49 g (yield: 92%) of 2-(2,2,2-trifluoroethyl)-1,2-benzisothiazoline-3-thioxo-1,1-dioxide as a yellow powder (melting point: 113 to 114°C).

$^1$H-NMR data ($CDCl_3$/TMS δ (ppm)): 4.76 (2H, q), 7.88 (3H, m), 8.31 (1H, d)

<Example 3>

Production of 2-ethoxycarbonyl-1,2-benzisothiazoline-3-thioxo-1,1-dioxide (compound No. 5)

(1) Production of 2-ethoxycarbonyl-1,2-benzisothiazoline-3-oxo-1,1-dioxide

4.0 g (21.8 mmole) of saccharin was dissolved in 20 ml of acetonitrile. Thereto was added 1.8 g (21.8 mmole) of sodium hydrogencarbonate and 2.5 g (23.0 mmole) of ethyl chlorocarbonate. The mixture was stirred for 3 hours under refluxing with heating, to give rise to a reaction. After confirmation of the completion of the reaction, the reaction mixture was cooled to room temperature and poured into water, followed by extraction with ethyl acetate. The resulting organic layer was washed with water and dried over anhydrous magnesium sulfate. The inorganic matter was removed by filtration. The filtrate was subjected to distillation under reduced pressure to remove the solvent. The residue was recrystallized from ethanol to obtain 5.5 g (yield: 99%) of 2-ethoxycarbonyl-1,2-benzisothiazoline-3-oxo-1,1-dioxide as a white powder (melting point: 132 to 134°C).

$^1$H-NMR data ($CDCl_3$/TMS δ (ppm)): 1.48 (3H, t), 4.55 (2H, q), 7.94 (3H, m), 8.17 (1H, d)

(2) Production of 2-ethoxycarbonyl-1,2-benzisothiazoline-3-thioxo-1,1-dioxide (compound No. 5)

2.0 g (7.8 mmole) of 2-ethoxycarbonyl-1,2-benzisothiazoline-3-oxo-1,1-dioxide was dissolved in 30 ml of xylene.

Thereto was added 1.74 g (7.8 mmole) of phosphorus pentasulfide. The mixture was stirred for 5 hours under refluxing with heating, to give rise to a reaction. After confirmation of the completion of the reaction, the reaction mixture was cooled to room temperature. The inorganic matter was removed by filtration. The filtrate was subjected to distillation under reduced pressure to remove the solvent. The residue was purified by silica gel column chromatography to obtain 0.2 g (yield: 9%) of 2-ethoxycarbonyl-1,2-benzisothiazoline-3-thioxo-1,1-dioxide as a dark pink powder (melting point: 118 to 121°C).
$^1H$-NMR data ($CDCl_3$/TMS δ (ppm)): 1.50 (3H, t), 4.58 (2H, q), 7.87 (3H, m), 8.28 (1H, d)

<Example 4>

Production of 2-(p-tolyl)-1,2-benzisothiazoline-3-thioxo-1,1-dioxide (compound No. 4)

(1) Production of 2-(p-tolyl)-1,2-benzisothiazoline-3-oxo-1,1-dioxide

1.0 g (5.5 mmole) of saccharin was dissolved in 30 ml of dichloromethane. Thereto were added 1.5 g (11.0 mmole) of 4-methylphenylboronic acid, 1.0 g (5.5 mmole) of copper (II) acetate and 0.86 g (11.0 mmole) of pyridine. The mixture was stirred at room temperature for 3 days to give rise to a reaction. After confirmation of the completion of the reaction, the reaction mixture was poured into water, followed by extraction with dichloromethane. The resulting organic layer was washed with water and dried over anhydrous magnesium sulfate. The inorganic matter was removed by filtration. The filtrate was subjected to distillation under reduced pressure to remove the solvent. The residue was purified by silica gel column chromatography to obtain 0.84 g (yield: 56%) of 2-(p-tolyl)-1,2-benzisothiazoline-3-oxo-1,1-dioxide as a white powder.
$^1H$-NMR data ($CDCl_3$/TMS δ (ppm)): 2.44 (3H, s), 7.36 (2H, d), 7.41 (2H, d), 7.90 (2H, m), 8.0 (1H, d), 8.16 (1H, d)

(2) Production of 2-(p-tolyl)-1,2-benzisothiazoline-3-thioxo-1,1-dioxide (compound No. 4)

0.8 g (2.9 mmole) of 2-(p-tolyl)-1,2-benzisothiazoline-3-oxo-1,1-dioxide was dissolved in 30 ml of xylene. Thereto was added 0.65 g (2.9 mmole) of phosphorus pentasulfide. The mixture was stirred at 100°C for 6 hours to give rise to a reaction. After confirmation of the completion of the reaction, the reaction mixture was cooled to room temperature. The inorganic matter was removed by filtration. The filtrate was subjected to distillation under reduced pressure to remove the solvent. The residue was purified by silica gel column chromatography to obtain 0.5 g (yield: 58%) of 2-(p-tolyl)-1,2-benzisothiazoline-3-thioxo-1,1-dioxide as an orange powder (melting point: 172 to 173°C).
$^1H$-NMR data ($CDCl_3$/TMS δ (ppm)): 2.46 (3H, s), 7.39 (4H, s), 7.87 (3H, m), 8.35 (1H, d)

<Example 5>

Production of 2-bromomethyl-1,2-benzisothiazoline-3-thioxo-1,1-dioxide (compound No. 44)

(1) Production of 2-bromomethyl-1,2-benzisothiazoline-3-oxo-1,1-dioxide

In 100 ml of acetic acid were dissolved 30.0 g (0.14 M) of 2-hydroxymethyl-1,2-benzisothiazoline-3-oxo-1,1-dioxide and 80 ml of an acetic acid solution containing 30% of hydrogen bromide. The mixture was stirred for 5 hours under refluxing with heating, to give rise to a reaction. The reaction mixture was poured into water. The resulting solid was collected by filtration and washed with water to obtain 31.2 g (yield: 81%) of 2-bromomethyl-1,2-benzisothiazoline-3-oxo-1,1-dioxide as a white powder.
$^1H$-NMR data ($CDCl_3$/TMS δ (ppm)): 5.53 (1H, s), 7.93 (3H, m), 8.14 (1H, d)

(2) Production of 2-bromomethyl-1,2-benzisothiazoline-3-thioxo-1,1-dioxide (compound No. 44)

1.0 g (3.6 mmole) of 2-bromomethyl-1,2-benzisothiazoline-3-oxo-1,1-dioxide was dissolved in 5 ml of xylene. Thereto was added 2.0 g (9.1 mmole) of phosphorus pentasulfide. The mixture was stirred for 5 hours under refluxing with heating, to give rise to a reaction. After confirmation of the completion of the reaction, the inorganic matter was removed by filtration. The filtrate was subjected to distillation under reduced pressure to remove the solvent. The residue was purified by silica gel column chromatography to obtain 0.4 g (yield: 36%) of 2-bromomethyl-1,2-benzisothiazoline-3-thioxo-1,1-dioxide as a yellow powder (melting point: 160 to 163°C).
$^1H$-NMR data ($CDCl_3$/TMS δ (ppm)): 5.79 (3H, t), 7.88 (3H, m), 8.29 (1H, d)

<Example 6>

Production of 2-benzoyloxymethyl-1,2-benzisothiazoline-3-thioxo-1,1-dioxide (compound No. 46)

In 20 ml of dichloromethane were dissolved 0.2 g (1.7 mmole) of benzoic acid, 0.4 g (1.4 mmole) of 2-bromomethyl-1,2-benzisothiazoline-3-thioxo-1,2-dioxide and 0.2 g (2.0 mmole) of triethylamine. The solution was stirred at room temperature for 3 hours to give rise to a reaction. After confirmation of the completion of the reaction, the reaction mixture was poured into water, followed by extraction with ethyl acetate. The resulting organic layer was washed with water and dried over anhydrous magnesium sulfate. The inorganic matter was removed by filtration. The filtrate was subjected to distillation under reduced pressure to remove the solvent. The residue was purified by silica gel column chromatography to obtain 0.1 g (yield: 20%) of 2-benzoyloxymethyl-1,2-benzisothiazoline-3-thioxo-1,1-dioxide as a yellow powder (melting point: 107 to 108°C).

$^1$H-NMR data ($CDCl_3$/TMS $\delta$ (ppm)): 6.39 (2H, s), 7.43 (2H, m), 7.57 (1H, m), 7.87 (3H, m), 8.05 (2H, d), 8.32 (1H, d)

<Example 7>

Production of 2-methoxy-1,2-benzisothiazoline-3-thioxo-1,1-dioxide (compound No. 43)

(1) Production of methyl 2-[(N-methoxyamino)sulfonyl]benzoate

1.8 g (21.3 mmole) of methoxyamine hydrochloride was suspended in 100 ml of chloroform. Thereto was dropwise added 5.4 g (53.8 mmole) of triethylamine at 0°C or lower. Further was dropwise added, at 0°C or lower, 5.0 g (21.3 mmole) of methyl 2-(chlorosulfonyl)benzoate dissolved in 30 ml of chloroform. The mixture was stirred at room temperature for 16 hours to give rise to a reaction. After confirmation of the completion of the reaction, the reaction mixture was poured into water, followed by extraction with chloroform. The resulting organic layer was washed with water and dried over anhydrous magnesium sulfate. The inorganic matter was removed by filtration. The filtrate was subjected to distillation under reduced pressure to remove the solvent. The resulting solid was washed with n-hexane to obtain 3.7 g (yield: 71%) of methyl 2-[(N-methoxyamino)sulfonyl]benzoate as a white powder.

(2) Production of 2-methoxy-1,2-benzisothiazoline-3-oxo-1,1-dioxide

3.7 g (15.1 mmole) of methyl 2-[(N-methoxyamino)sulfonyl]benzoate was placed in a reactor and stirred at 170°C for 3 hours to give rise to a reaction. After confirmation of the completion of the reaction, the reaction mixture was poured into water, followed by extraction with ethyl acetate. The resulting organic layer was washed with water and dried over anhydrous magnesium sulfate. The inorganic matter was removed by filtration. The filtrate was subjected to distillation under reduced pressure to remove the solvent. The residue was purified by silica gel column chromatography to obtain 0.5 g ((yield: 19%) of 2-methoxy-1,2-benzisothiazoline-3-oxo-1,1-dioxide as a white powder.

$^1$H-NMR data ($CDCl_3$/TMS $\delta$ (ppm)): 4.28 (3H, s), 7.91 (3H, m), 8.06 (1H, d)

(3) Production of 2-methoxy-1,2-benzisothiazoline-3-thioxo-1,1-dioxide (compound No. 43)

0.5 g (2.3 mmole) of 2-methoxy-1,2-benzisothiazoline-3-thioxo-1,1-dioxide was dissolved in 5 ml of xylene. Thereto was added 1.3 g (5.8 mmole) of phosphorus pentasulfide. The mixture was stirred for 2 hours under refluxing with heating, to give rise to a reaction. After confirmation of the completion of the reaction, the inorganic matter was removed by filtration. The filtrate was subjected to distillation under reduced pressure to remove the solvent. The residue was purified by silica gel column chromatography to obtain 90 mg (yield: 17%) of 2-methoxy-1,2-benzisothiazoline-3-thioxo-1,1-dioxide as a yellow powder (melting point: 172 to 175°C).

$^1$H-NMR data ($CDCl_3$/TMS $\delta$ (ppm)): 3.54 (3H, s), 7.84 (3H, m), 8.27 (1H, d)

The structural formulas and physical properties of the present application compounds [I] synthesized in the above Examples or based on the Examples are shown in Table 13 and Table 14. The symbols in these tables have the same meanings as given before.

Table 13

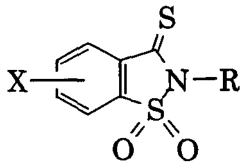

| Compound No. | R | X | Melting point (°C) |
|---|---|---|---|
| 1 | $CH_2CF_3$ | H | 113-114 |
| 2 | $CH_2OMe$ | H | 94-95 |
| 3 | $CH_2OEt$ | H | 76-78 |
| 4 | Ph(4-Me) | H | 172-173 |
| 5 | C(=O)OEt | H | 118-121 |
| 6 | C(=O)OPr | H | 98-99 |
| 7 | $C(=O)OCH_2CH=CH_2$ | H | 113-114 |
| 8 | $C(=O)OCH_2C{\equiv}CH$ | H | 134-136 |
| 9 | C(=O)OBu-i | H | 100-101 |
| 10 | C(=O)OPh(4-Cl) | H | 155-157 |
| 11 | Pr-i | H | 1.6231 |
| 12 | $CH_2CH=CH_2$ | H | 76-77 |
| 13 | $CH_2C{\equiv}CH$ | H | 128-129 |
| 14 | $CH_2CH_2CF_3$ | H | 78-79 |
| 15 | $CH_2CH_2Cl$ | H | 57-58 |
| 16 | $CH_2OPr$ | H | 1.5762 |
| 17 | $CH_2SMe$ | H | 119-120 |
| 18 | $CH_2CO_2Me$ | H | 131-132 |
| 19 | $CH_2CN$ | H | 168-169 |
| 20 | $CH_2NO_2$ | H | 132-133 |
| 21 | $CH_2Ph$(4-Cl) | H | 138-139 |
| 22 | COMe | H | 153-155 |
| 23 | COOMe | H | 180-181 |
| 24 | COOPr-i | H | 109-110 |
| 25 | COOPh | H | 180-182 |
| 26 | $CH_2CH_2C(Me)=CF_2$ | H | 52-53 |
| 27 | COEt | H | 110-113 |
| 28 | COBu | H | 96-97 |
| 29 | Pn-c | H | 97-99 |
| 30 | $COOCH_2CH_2Cl$ | H | 107-109 |
| 31 | COOBu | H | 81-82 |

Table 14

| Compound No. | R | X | Melting point (°C) |
|---|---|---|---|
| 32 | $CH_2CO_2Et$ | H | 131-132 |
| 33 | $CH_2C(=S)OEt$ | H | 61-62 |
| 34 | $CH_2CO_2Ph$ | H | 107-109 |
| 35 | COOBu-t | H | 60-61 |
| 36 | $COO(CH_2)_5CH_3$ | H | 48-49 |
| 37 | $COO(CH_2)_7CH_3$ | H | 40-42 |
| 38 | $COO(CH_2)_9CH_3$ | H | 49-50 |

Table continued

| Compound No. | R | X | Melting point (°C) |
|---|---|---|---|
| 39 | $CH_2CH_2NO_2$ | H | 130-131 |
| 40 | $CH(CH_3)CH_2NO_2$ | H | 93-94 |
| 41 | $CH_2CONMe_2$ | H | 183-184 |
| 42 | $CH(COOEt)_2$ | H | 110-111 |
| 43 | $OCH_3$ | H | 172-175 |
| 44 | $CH_2Br$ | H | 160-163 |
| 45 | $CH_2Cl$ | H | 181-182 |
| 46 | $CH_2OC(=O)Ph$ | H | 107-108 |
| 47 | $CH_2OC(=O)(2,6\text{-}Cl_2\text{-pyridin-4-yl})$ | H | 176-178 |
| 48 | $CH_2SCN$ | H | 145-147 |
| 49 | 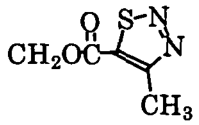<br>$CH_3$ | H | 116-117 |
| 50 | $CH_2CH=CHCl$ | H | 1.6433 |
| 51 | $COOCH_2(2,6\text{-}Cl_2\text{-pyridin-4-yl})$ | H | 187-188 |
| 52 | $CH_2OCH_2(2,6\text{-}Cl_2\text{-pyridin-4-yl})$ | H | 129-131 |
| 53 | $CH_2CH_2OMe$ | H | 97-99 |
| 55 | COOEt | 5-Cl | 151-152 |
| 54 | $CH_2(2,6\text{-}Cl_2\text{-pyridin-4-yl})$ | H | 185-186 |
| 56 | $CH_2SCH_2(2,6\text{-}Cl_2\text{-pyridin-4-yl})$ | H | 169-170 |

As to the compound Nos. 11, 16 and 50, the $^1H$-NMR data [$CDCl_3$/TMS δ (ppm)] are shown below.

Compound No. 11: 1.70 (6H, d), 5.24 (1H, m), 7.76 (3H, m), 8.24 (1H, d)
Compound No. 16: 0.93 (3H, t), 1.66 (2H, m), 3.67 (2H, t), 5.61 (2H, s), 7.81 (3H, m), 8.30 (1H, d)
Compound No. 50: 4.95 (2H, d), 5.99 (1H, q), 6.32 (1H, d), 7.78 (3H, m), 8.28 (1H, d)

Next, there are mentioned representative examples of the plant disease control agent and the pest control agent both for agricultural and horticultural use, of the present invention, and specific production methods thereof are described. In the following description, "%" refers to % by weight.

<Example 8> Production Example 1 (dust)

There were uniformly mixed and ground a present application compound (compound No. 2) (2%), diatomaceous earth (5%) and clay (93%) to obtain a dust.

<Example 9> Production Example 2 (wettable powder)

There were uniformly mixed and ground a present application compound (compound No. 3) (50%), diatomaceous earth (45%), sodium dinaphthylmethanedisulfonate (2%) and sodium lignosulfonate (3%) to obtain a wettable powder.

<Example 10> Production Example 3 (emulsifiable concentrate)

A present application compound (compound No. 5) (30%), cyclohexanone (20%), polyoxyethylene alkylaryl ether (11%), calcium alkylbenzenesulfonate (4%) and methylnaphthalene (35%) were made into a uniform solution to obtain an emulsifiable concentrate.

<Example 11> Production Example 4 (granule)

There were uniformly mixed and ground a present invention compound (compound No. 7) (24%), sodium lauryl sulfate (2%), sodium lignosulfonate (5%), carboxymethyl cellulose (2%) and clay (67%). To this mixture was added water (20% of the mixture), followed by kneading. The kneaded product was passed through an extrusion granulator to obtain grains of 14 to 32 meshes. The grains were dried to obtain a granule.

<Example 12> Production Example 5 (granule)

There were uniformly mixed, in a high-speed stirrer, a present application compound (compound No. 8) (26%), sodium alkylbenzenesulfonate (0.4%), $\alpha$-starch (3%) and clay (70.6%). An appropriate amount of water was added, followed by kneading. The kneaded product was passed trough a basket type granulator having a screen of 1.0 mm opening diameter. The resulting grains were allowed to stand at 60°C for drying, to obtain a base material. This base material (82%) was mixed with an acrylic resin (6%), a urethane resin (3%) and clay (9%) to obtain a release-controlled granule containing 21.3% by mass of a present application compound (compound No. 8).
Next, there are specifically described the effects of plant disease control agents for agricultural and horticultural use, of the present invention, by way of Test Examples.

<Test Example 1> Test for preventive effect on rice blast

15 rice seeds (variety: Aichi Asahi) were sown in each of unglazed pots of 7 cm in diameter and seedlings were grown in a greenhouse. When the growth of seedlings reached a stage at which the fourth leaf developed completely, one kind of the wettable powders produced based on the Production Example 2 was diluted with water so as to give an active ingredient concentration of 500 ppm, and the diluted powder was sprayed on the seedlings of one of the pots in an amount of 10 ml per pot. After the seedlings were air-dried, a suspension of conidia of Pyricularia oryzae was sprayed (for inoculation) on the rice seedlings. Immediately, each pot was placed in a wet house of 25°C for 24 hours. Then, each pot was transferred into a greenhouse and, after 5 days from the inoculation, the number of lesions of the fourth leaf was examined. A preventive value was calculated using the following calculation expression and the wettable powder used was evaluated according to the standard of Table 15. The results of evaluation are shown in Table 16.
Calculation expression 1

Preventive value =
[1-(the number of lesions of treated-plot)/
(the number of lesions of untreated-plot)] x 100

Table 15

| Evaluation | Preventive value |
|---|---|
| A | 100 |
| B | Less than 100 to 80.0 or more |
| C | Less than 80.0 to 50.0 or more |

Table continued

| Evaluation | Preventive value |
|---|---|
| D | Less than 50.0 |

Table 16

| Compound No. | Evaluation |
|---|---|
| 2 | B |
| 3 | B |
| 5 | B |
| 6 | A |
| 7 | B |
| 8 | B |
| 9 | B |
| 27 | B |
| 30 | B |
| 39 | B |
| 40 | B |
| 43 | B |
| 44 | B |
| 45 | A |
| 46 | B |
| 48 | B |
| 49 | B |

<Test Example 2> Test for control effect on rice blast when applied to submerged application

Young paddy rice seedlings of 1.5 leaf stage (variety: Aichi Asahi) were transplanted into each of white porcelain pots of 9 cm in diameter (each three stems were planted in four places of the pot), and they were grown in a greenhouse. At a 2.5 leaf stage, one kind of the wettable powder produced based on the Production Example 2 was applied onto the water surface of one of the pots so that the active ingredient of the wettable powder became 1,000 g per 10 ares. After 10 days from the application, a suspension of conidia of Pyricularia oryzae was sprayed (for inoculation) on the seedlings of the pot. Immediately, the pot was placed in a wet house of 25°C for 24 hours. Then, the pot was transferred into a greenhouse and, after 5 days from the inoculation, the number of lesions of the upper young leaf at the time of inoculation was examined. A preventive value was calculated from the above calculation expression. The wettable powder used was evaluated based on the standard shown in Table 15. The results of evaluation are shown in Table 17.

Table 17

| Compound No. | Evaluation |
|---|---|
| 2 | B |
| 3 | B |
| 5 | B |
| 6 | B |

Table continued

| Compound No. | Evaluation |
|---|---|
| 7 | B |
| 8 | B |
| 9 | B |
| 10 | B |
| 11 | B |
| 17 | B |
| 19 | B |
| 20 | B |
| 22 | B |
| 23 | B |
| 24 | B |
| 25 | B |
| 31 | B |
| 39 | B |
| 40 | B |
| 43 | B |
| 44 | B |
| 45 | B |
| 46 | B |
| 47 | B |
| Probenazole (control compound) | B |

<Test Example 3> Test for control effect on rice blast when applied to seedling-raising box

An artificial soil was filled in each of rice seedling-raising boxes (30 cm x 60 cm x 3 cm). 180 g (as dried weight) per box, of seed rice (variety: Aichi Asahi) was sown. After 3 weeks therefrom, one of the granules produced based on the production Example 4 was uniformly applied to one of the seedling-raising boxes so that the amount of the active ingredient applied became 12 g per box. After 4 hours therefrom, 5 stems of the rice seedlings were taken out together with the artificial soil and transplanted into a 1/10,000 are Wagner pot. After 40 days from the application, a suspension of conidia of Pyricularia oryzae was sprayed for inoculation. Immediately, the pot was placed in a wet house of 25°C for 24 hours. Then, the pot was transferred into a greenhouse and, after 6 days from the inoculation, the number of lesions of the upper young leaf at the time of inoculation was examined. A preventive value was calculated from the above calculation expression. The wettable powder used was evaluated based on the standard shown in Table 15. The results of evaluation are shown in Table 18.

Table 18

| Compound No. | Evaluation |
|---|---|
| 2 | B |
| 3 | B |
| 5 | B |
| 47 | B |
| Probenazole (control compound) | B |

<Test Example 4> Test for control effect on Septoria leaf spot of wheat

In each of plastic pots of 6 cm in diameter were sown 10 wheat seeds (variety: Nohrin No. 61). They were allowed to germinate in a greenhouse. When wheat seedlings of 2 leaves emerged, one kind of the wettable powders produced based on the Production Example 2 was diluted with water so as to give an active ingredient concentration of 500 ppm and the resulting solution was applied to the seedlings in an amount of 10 ml per pot. After air-drying, the seedlings were inoculated with pycnospores of Septoria nodorum and the pot was placed in a greenhouse. After 10 days from the inoculation, the total diseased area of all the first leaves in the pot was examined. The wettable powder used was evaluated according to the standard of Table 19. The results of evaluation are shown in Table 20.

Table 19

| Evaluation | Diseased area |
|---|---|
| A | No infection. |
| B | Less than 25% of untreated plot |
| C | 25% or more to less than 50%, of untreated plot |
| D | 50% or more of untreated plot |

Table 20

| Compound No | Evaluation |
|---|---|
| 2 | A |
| 5 | B |
| 6 | B |
| 7 | B |
| 8 | A |
| 9 | B |
| 10 | B |
| 29 | B |
| 30 | B |
| 31 | B |
| 39 | B |
| 40 | B |
| 44 | B |
| 45 | A |
| 48 | B |
| 50 | B |

<Test Example 5> Test for control effect on powdery mildew of wheat

In each of plastic pots of 6 cm in diameter were sown 10 wheat seeds (variety: Nohrin No. 61). They were allowed to germinate in a greenhouse. When wheat seedlings of 2 leaves emerged, one kind of the wettable powders produced based on the Production Example 2 was diluted with water so as to give an active ingredient concentration of 500 ppm and the resulting solution was applied to the seedlings in an amount of 10 ml per pot. After 7 days from the application, the seedlings were inoculated, by spraying, with conidia of Erysiphe graminis. After 8 days from the inoculation, the total infected area of all the first leaves in the pot was examined. The wettable powder used was evaluated

according to the standard of Table 19. The results of evaluation are shown in Table 21.
Table 21

| Compound No. | Evaluation |
|---|---|
| 2 | B |
| 5 | A |
| 8 | B |

<Test Example 6> Test for control effect on downy mildew of cucumber

10 cucumber seeds (variety: sagami hanjiro) were sown in each of vinyl chloride resin made pots of 9 cm x 9 cm and were allowed to germinate in a greenhouse for 7 days. The resulting cucumber young seedlings which reached a cotyledon stage were used as a sample plant. One kind of the wettable powders produced based on the Production Example 2 was diluted with water so as to give an active ingredient concentration of 500 ppm and the resulting solution was sprayed onto the sample plant in an amount of 15 ml per pot. The plant was air-dried and inoculated, by spraying, with a suspension of conidia of Pseudoperonospora cubensis. Immediately, the pot was placed in a wet house of 20°C for 24 hours. Then, the pot was transferred into a greenhouse. 7 days later, the total diseased area of all the cotyledons in the pot was examined. The wettable powder used was evaluated according to the standard of Table 19. The results of evaluation are shown in Table 22.
Table 22

| Compound No. | Evaluation |
|---|---|
| 2 | B |
| 6 | B |
| 9 | B |
| 39 | A |
| 42 | B |
| 48 | B |
| 50 | B |
| 53 | B |

Next, there are specifically described the effects of pest control agents for agricultural and horticultural use, of the present invention, by way of Test Examples.

<Test Example 7> Test for control of brown planthopper

A wettable powder produced based on the Production Example 2 was diluted with water so as to give an active ingredient concentration of 500 ppm. In the resulting solution was immersed germinated unhulled rice. Then, the germinated unhulled rice was placed in a 60-ml plastic cup. Thereinto were released 10 4th-instar larvae of brown planthopper. The cup was capped and placed in an incubator of 25°C. After 6 days therefrom, the number of surviving larvae was counted and the mortality of larvae was determined using the following calculation expression.
Calculation expression 2

Mortality = [(10 - the number of surviving larvae)/10] x 100

<Test Example 8> Test for control of two-spotted spider mite

A wettable powder produced based on the Production Example 2 was diluted with water so as to give an active ingredient concentration of 500 ppm. In the resulting solution were immersed soybean seedlings beforehand infested with two-spotted spider mite adults. Then, the soybean seedlings were air-dried and placed in an incubator of 25°C. 13 days later, the number of surviving adults was examined. A preventive value was determined using the following calculation expression 3.

Calculation expression 3

Preventive value =

{1 - [(the number of adults before treatment, of untreated plot)/(the number of adults before treatment, of treated plot)] x [(the number of adults on examination day, of treated plot)/(the number of adults on examination day, of untreated plot)]} x 100

<Test Example 9> Test for root-knot nematode

5% of a test compound was dissolved in N,N-dimethylformamide containing 1 % of Tween 20, to prepare an emulsifiable concentrate. This emulsifiable concentrate was diluted with water so as to give an active ingredient concentration of 20 ppm. 0.5 ml of the resulting solution was mixed with 0.5 ml of a suspension containing about 100 second-stage larvae of southern root-knot nematode. The mixture was placed in an incubator of 25°C. 2 days later, the number of surviving southern root-knot nematode larvae was examined using a microscope. Nemastatic degree (%) was determined using the following calculation expression.

Calculation expression 4

Nemastatic degree (%) =

{[(the number of surviving nematode larvae of untreated plot) - (the number of surviving nematode larvae of treated plot)]/(the number of surviving nematode larvae of untreated plot)} x 100

As representative examples of the compound showing, in the above test, a nemastatic activity of "nemastatic degree = 90% or more", there are mentioned compounds No. 6, 23, 31 and 33.

Industrial Applicability

The present application compound represented by the general formula [I] is highly effective in controlling agricultural or horticultural plant diseases such as rice blast, Septoria leaf spot of wheat, powdery mildew of wheat, downy mildew of cucumber and the like; and is highly effective in controlling a wide range of pests such as pests of Hemiptera, pests of Lepidoptera, pests of Coleoptera, pests of Diptera, pests of Hymenoptera, pests of Orthoptera, pests of Isoptera, pests of Thysanoptera, spider mites, plant-parasitic nematodes and the like. The compound is effective

also in controlling pests having resistance. Furthermore, the compound is excellent in residual activity and rain resistance without causing chemical damage to crops. Therefore, the compound is useful as a plant disease control agent for agricultural or horticultural use, or as a pest control agent for agricultural or horticultural use.

## Claims

1. A plant disease control agent for agricultural or horticultural use, containing, as an active ingredient, a 1,2-benzisothiazoline-3-thioxo-1,1-dioxide derivative represented by the following general formula [I] or a salt thereof:

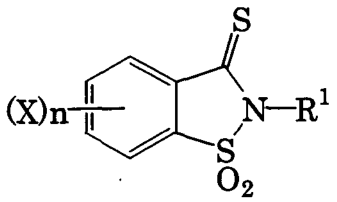

[ I ]

{wherein $R^1$ is $C_1$-$C_{12}$ alkyl group, $C_2$-$C_6$ alkenyl group, $C_2$-$C_6$ alkynyl group, $C_1$-$C_6$ alkoxy group, $C_3$-$C_6$ cycloalkyl group, $C_1$-$C_6$ haloalkyl group, $C_2$-$C_6$ haloalkenyl group, $C_3$-$C_6$ cycloalkyl $C_1$-$C_6$ alkyl group, $C_1$-$C_6$ alkoxy $C_1$-$C_6$ alkyl group, $C_1$-$C_6$ alkylthio $C_1$-$C_6$ alkyl group, -C(=Y)-$R^2$ group, -C(=Y)-$Y^1$-$R^3$ group, -W-C (=O) -$R^4$ group, -W-(C(=O)-$R^4$)$_2$ group, -W-$Y^1$-W-$R^{12}$ group, -W-$Y^1$-(C(=Y))$_m$-$R^{12}$ group, -W-$R^{12}$ group, -$Y^1$-W-$R^{12}$ group, -W-$Y^1$-N=C$R^{12}R^{14}$ group, hydroxy $C_1$-$C_6$ alkyl group, cyano $C_1$-$C_6$ alkyl group, cyanothio $C_1$-$C_6$ alkyl group, isothiocyano $C_1$-$C_6$ alkyl group, nitro $C_1$-$C_6$ alkyl group, $NR^6R^7$ $C_1$-$C_6$ alkyl group, phenyl $C_1$-$C_6$ alkyl group (this phenyl $C_1$-$C_6$ alkyl group may be substituted with halogen atom, nitro group, cyano group, $C_1$-$C_6$ alkyl group, $C_1$-$C_6$ alkoxy group, $C_1$-$C_6$ haloalkyl group, $C_1$-$C_6$ haloalkoxy group or $NR^6R^7$ group), phenyl group (this phenyl group may be substituted with halogen atom, nitro group, cyano group, $C_1$-$C_6$ alkyl group, $C_1$-$C_6$ alkoxy group, $C_1$-$C_6$ haloalkyl group, $C_1$-$C_6$ haloalkoxy group or $NR^6R^7$ group), or $C_3$-$C_{10}$ heterocyclic group having at least one hetero atom which is selected from oxygen atom, sulfur atom and nitrogen atom and which may be the same or different from each other (this heterocyclic group may be substituted with halogen atom, nitro group, cyano group, $C_1$-$C_6$ alkyl group, $C_1$-$C_6$ alkoxy group, $C_1$-$C_6$ haloalkyl group, $C_1$-$C_6$ haloalkoxy group or $NR^6R^7$ group); X is halogen atom, nitro group, cyano group, $C_1$-$C_6$ alkyl group, $C_1$-$C_6$ alkoxy group, $C_1$-$C_6$ haloalkyl group, $C_1$-$C_6$ haloalkoxy group, or $NR^6R^7$ group;

$R^2$ is $C_1$-$C_6$ alkyl group, $C_1$-$C_6$ alkenyl group, -W-$R^{12}$ group, $C_1$-$C_6$ haloalkyl group, $NR^{15}R^{16}$ group, or phenyl group (this phenyl group may be substituted with halogen atom, nitro group, cyano group, $C_1$-$C_6$ alkyl group, $C_1$-$C_6$ alkoxy group, $C_1$-$C_6$ haloalkyl group, $C_1$-$C_6$ haloalkoxy group or $NR^8R^9$ group) ;

$R^3$ is $C_1$-$C_{12}$ alkyl group, $C_2$-$C_6$ alkenyl group, $C_2$-$C_6$ alkynyl group, $C_3$-$C_6$ cycloalkyl group, $C_1$-$C_6$ haloalkyl group, $C_1$-$C_6$ alkoxy $C_1$-$C_6$ alkyl group, -W-$R^{12}$ group, benzyl group (this benzyl group may be substituted with halogen atom, nitro group, cyano group, $C_1$-$C_6$ alkyl group, $C_1$-$C_6$ alkoxy group, $C_1$-$C_6$ haloalkyl group, $C_1$-$C_6$ haloalkoxy group or $NR^8R^9$ group), or phenyl group (this phenyl group may be substituted with halogen atom, nitro group, cyano group, $C_1$-$C_6$ alkyl group, $C_1$-$C_6$ alkoxy group, $C_1$-$C_6$ haloalkyl group, $C_1$-$C_6$ haloalkoxy group or $NR^8R^9$ group);

$R^4$ is $C_1$-$C_6$ alkyl group, $C_1$-$C_6$ haloalkyl group, phenyl group (this phenyl group may be substituted with halogen atom, nitro group, cyano group, $C_1$-$C_6$ alkyl group, $C_1$-$C_6$ alkoxy group, $C_1$-$C_6$ haloalkyl group, $C_1$-$C_6$ haloalkoxy group or $NR^8R^9$ group), $NR^6R^7$ group or $Y^2R^5$ group ($Y^2$ is oxygen atom or sulfur atom);

$R^5$ is $C_1$-$C_{12}$ alkyl group, $C_2$-$C_6$ alkenyl group, $C_2$-$C_6$ alkynyl group, $C_3$-$C_6$ cycloalkyl group, $C_1$-$C_6$ haloalkyl group, $C_1$-$C_6$ alkoxy $C_1$-$C_6$ alkyl group, benzyl group (this benzyl group may be substituted with halogen atom, nitro group, cyano group, $C_1$-$C_6$ alkyl group, $C_1$-$C_6$ alkoxy group, $C_1$-$C_6$ haloalkyl group, $C_1$-$C_6$ haloalkoxy group or $NR^8R^9$ group), or phenyl group (this phenyl group may be substituted with halogen atom, nitro group, cyano group, $C_1$-$C_6$ alkyl group, $C_1$-$C_6$ alkoxy group, $C_1$-$C_6$ haloalkyl group, $C_1$-$C_6$ haloalkoxy group or $NR^8R^9$ group);

$R^6$ and $R^7$ are each independently hydrogen atom, $C_1$-$C_6$ alkyl group, $C_2$-$C_6$ alkenyl group, -W-$R^{12}$ group, or phenyl group (this phenyl group may be substituted with halogen atom, nitro group, cyano group, $C_1$-$C_6$ alkyl group, $C_1$-$C_6$ alkoxy group, $C_1$-$C_6$ haloalkyl group, $C_1$-$C_6$ haloalkoxy group or $NR^8R^9$ group);

$R^8$ and $R^9$ are each independently hydrogen atom or $C_1$-$C_6$ alkyl group;

$R^{12}$ is phenyl group (this phenyl group may be substituted with halogen atom, nitro group, cyano group, $C_1$-$C_6$ alkyl group, $C_1$-$C_6$ alkoxy group, $C_1$-$C_6$ haloalkyl group, $C_1$-$C_6$ haloalkoxy group or $NR^8R^9$ group), or $C_3$-$C_{10}$ heterocyclic group having at least one hetero atom which is selected from oxygen atom, sulfur atom and nitrogen atom and which may be the same or different from each other (this heterocyclic group may be substituted with

halogen atom, nitro group, cyano group, $C_1$-$C_6$ alkyl group, $C_1$-$C_6$ alkoxy group, $C_1$-$C_6$ haloalkyl group, $C_1$-$C_6$ haloalkoxy group or $NR^6R^7$ group);
$R^{13}$ is $C_3$-$C_{10}$ heterocyclic group having at least one hetero atom which is selected from oxygen atom, sulfur atom and nitrogen atom and which may be the same or different from each other (this heterocyclic group may be substituted with halogen atom, nitro group, cyano group, $C_1$-$C_6$ alkyl group, $C_{1-}C_6$ alkoxy group, $C_1$-$C_6$ haloalkyl group, $C_1$-$C_6$ haloalkoxy group or $NR^6R^7$ group);
$R^{14}$ is hydrogen atom or $C_1$-$C_6$ alkyl group;
$R^{15}$ and $R^{16}$ are each independently $C_1$-$C_6$ alkyl group or -W-$R^{13}$ group (when either one of $R^{15}$ and $R^{16}$ is -W-$R^{13}$ group, the other may be hydrogen atom);
n is an integer of 0 to 4;
m is an integer of 0 or 1;
Y is oxygen atom or sulfur atom;
$Y^1$ is oxygen atom or sulfur atom; and
W is $C_1$-$C_4$ alkylene group or $C_1$-$C_4$ alkenylene group}.

**2.** A pest control agent for agricultural or horticultural use, containing, as an active ingredient, a 1,2-benzisothiazoline-3-thioxo-1,1-dioxide derivative represented by the following general formula [I] or a salt thereof:

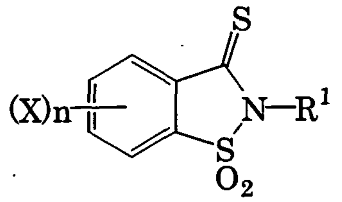

[I]

{wherein $R^1$ is $C_1$-$C_{12}$ alkyl group, $C_2$-$C_6$ alkenyl group, $C_2$-$C_6$ alkynyl group, $C_1$-$C_6$ alkoxy group, $C_3$-$C_6$ cycloalkyl group, $C_{1-}C_6$ haloalkyl group, $C_2$-$C_6$ haloalkenyl group, $C_3$-$C_6$ cycloalkyl $C_1$-$C_6$ alkyl group, $C_1$-$C_6$ alkoxy $C_1$-$C_6$ alkyl group, $C_1$-$C_6$ alkylthio $C_1$-$C_6$ alkyl group, -C(=Y)-$R^2$ group, -C(=Y)-$Y^1$-$R^3$ group, -W-C (=O) -$R^4$ group, -W-(C (=O)-$R^4$)$_2$ group, -W-$Y^1$-W-$R^{12}$ group, -W-$Y^1$-(C(=Y))$_m$-$R^{12}$ group, -W-$R^{12}$ group, -$Y^1$-W-$R^{12}$ group, -W-$Y^1$-N=C$R^{12}R^{14}$ group, hydroxy $C_1$-$C_6$ alkyl group, cyano $C_1$-$C_6$ alkyl group, cyanothio $C_1$-$C_6$ alkyl group, isothiocyano $C_1$-$C_6$ alkyl group, nitro $C_1$-$C_6$ alkyl group, $NR^6R^7$ $C_1$-$C_6$ alkyl group, phenyl $C_1$-$C_6$ alkyl group (this phenyl $C_1$-$C_6$ alkyl group may be substituted with halogen atom, nitro group, cyano group, $C_1$-$C_6$ alkyl group, $C_1$-$C_6$ alkoxy group, $C_1$-$C_6$ haloalkyl group, $C_1$-$C_6$ haloalkoxy group or $NR^6R^7$ group), phenyl group (this phenyl group may be substituted with halogen atom, nitro group, cyano group, $C_1$-$C_6$ alkyl group, $C_1$-$C_6$ alkoxy group, $C_1$-$C_6$ haloalkyl group, $C_1$-$C_6$ haloalkoxy group or $NR^6R^7$ group), or $C_{3-}C_{10}$ heterocyclic group having at least one hetero atom which is selected from oxygen atom, sulfur atom and nitrogen atom and which may be the same or different from each other (this heterocyclic group may be substituted with halogen atom, nitro group, cyano group, $C_1$-$C_6$ alkyl group, $C_1$-$C_6$ alkoxy group, $C_1$-$C_6$ haloalkyl group, $C_1$-$C_6$ haloalkoxy group or $NR^6R^7$ group); X is halogen atom, nitro group, cyano group, $C_1$-$C_6$ alkyl group, $C_1$-$C_6$ alkoxy group, $C_1$-$C_6$ haloalkyl group, $C_1$-$C_6$ haloalkoxy group, or $NR^6R^7$ group;
$R^2$ is $C_1$-$C_6$ alkyl group, $C_1$-$C_6$ alkenyl group, -W-$R^{12}$ group, $C_{1-}C_6$ haloalkyl group, $NR^{15}R^{16}$ group, or phenyl group (this phenyl group may be substituted with halogen atom, nitro group, cyano group, $C_1$-$C_6$ alkyl group, $C_1$-$C_6$ alkoxy group, $C_1$-$C_6$ haloalkyl group, $C_1$-$C_6$ haloalkoxy group or $NR^8R^9$ group);
$R^3$ is $C_1$-$C_{12}$ alkyl group, $C_2$-$C_6$ alkenyl group, $C_2$-$C_6$ alkynyl group, $C_3$-$C_6$ cycloalkyl group, $C_1$-$C_6$ haloalkyl group, $C_1$-$C_6$ alkoxy $C_1$-$C_6$ alkyl group, -W-$R^{12}$ group, benzyl group (this benzyl group may be substituted with halogen atom, nitro group, cyano group, $C_1$-$C_6$ alkyl group, $C_1$-$C_6$ alkoxy group, $C_{1-}C_6$ haloalkyl group, $C_1$-$C_6$ haloalkoxy group or $NR^8R^9$ group), or phenyl group (this phenyl group may be substituted with halogen atom, nitro group, cyano group, $C_1$-$C_6$ alkyl group, $C_{1-}C_6$ alkoxy group, $C_1$-$C_6$ haloalkyl group, $C_1$-$C_6$ haloalkoxy group or $NR^8R^9$ group);
$R^4$ is $C_1$-$C_6$ alkyl group, $C_1$-$C_6$ haloalkyl group, phenyl group (this phenyl group may be substituted with halogen atom, nitro group, cyano group, $C_1$-$C_6$ alkyl group, $C_1$-$C_6$ alkoxy group, $C_1$-$C_6$ haloalkyl group, $C_1$-$C_6$ haloalkoxy group or $NR^8R^9$ group), $NR^6R^7$ group or $Y^2R^5$ group ($Y^2$ is oxygen atom or sulfur atom); $R^5$ is $C_1$-$C_{12}$ alkyl group, $C_2$-$C_6$ alkenyl group, $C_2$-$C_6$ alkynyl group, $C_3$-$C_6$ cycloalkyl group, $C_1$-$C_6$ haloalkyl group, $C_1$-$C_6$ alkoxy $C_1$-$C_6$ alkyl group, benzyl group (this benzyl group may be substituted with halogen atom, nitro group, cyano group, $C_1$-$C_6$ alkyl group, $C_1$-$C_6$ alkoxy group, $C_1$-$C_6$ haloalkyl group, $C_1$-$C_6$ haloalkoxy group or $NR^8R^9$ group), or phenyl group (this phenyl group may be substituted with halogen atom, nitro group, cyano group, $C_1$-$C_6$ alkyl group, $C_1$-$C_6$ alkoxy group, $C_{1-}C_6$ haloalkyl group, $C_1$-$C_6$ haloalkoxy group or $NR^8R^9$ group);

$R^6$ and $R^7$ are each independently hydrogen atom, $C_1$-$C_6$ alkyl group, $C_2$-$C_6$ alkenyl group, -W-$R^{12}$ group, or phenyl group (this phenyl group may be substituted with halogen atom, nitro group, cyano group, $C_1$-$C_6$ alkyl group, $C_1$-$C_6$ alkoxy group, $C_1$-$C_6$ haloalkyl group, $C_1$-$C_6$ haloalkoxy group or $NR^8R^9$ group);
$R^8$ and $R^9$ are each independently hydrogen atom or $C_1$-$C_6$ alkyl group;
$R^{12}$ is phenyl group (this phenyl group may be substituted with halogen atom, nitro group, cyano group, $C_1$-$C_6$ alkyl group, $C_1$-$C_6$ alkoxy group, $C_1$-$C_6$ haloalkyl group, $C_1$-$C_6$ haloalkoxy group or $NR^8R^9$ group), or $C_3$-$C_{10}$ heterocyclic group having at least one hetero atom which is selected from oxygen atom, sulfur atom and nitrogen atom and which may be the same or different from each other (this heterocyclic group may be substituted with halogen atom, nitro group, cyano group, $C_1$-$C_6$ alkyl group, $C_1$-$C_6$ alkoxy group, $C_1$-$C_6$ haloalkyl group, $C_1$-$C_6$ haloalkoxy group or $NR^6R^7$ group);
$R^{13}$ is $C_3$-$C_{10}$ heterocyclic group having at least one hetero atom which is selected from oxygen atom, sulfur atom and nitrogen atom and which may be the same or different from each other (this heterocyclic group may be substituted with halogen atom, nitro group, cyano group, $C_1$-$C_6$ alkyl group, $C_1$-$C_6$ alkoxy group, $C_1$-$C_6$ haloalkyl group, $C_1$-$C_6$ haloalkoxy group or $NR^6R^7$ group);
$R^{14}$ is hydrogen atom or $C_1$-$C_6$ alkyl group;
$R^{15}$ and $R^{16}$ are each independently $C_1$-$C_6$ alkyl group or -W-$R^{13}$ group (when either one of $R^{15}$ and $R^{16}$ is -W-$R^{13}$ group, the other may be hydrogen atom);
n is an integer of 0 to 4;
m is an integer of 0 or 1;
Y is oxygen atom or sulfur atom;
$Y^1$ is oxygen atom or sulfur atom; and
W is $C_1$-$C_4$ alkylene group or $C_1$-$C_4$ alkenylene group}.

**3.** A 1,2-benzisothiazoline-3-thioxo-1,1-dioxide derivative represented by the following general formula [I] or a salt thereof:

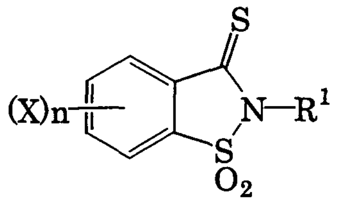

[I]

{wherein $R^1$ is $C_3$-$C_{12}$ alkyl group, $C_2$-$C_6$ alkenyl group, $C_2$-$C_6$ alkynyl group, $C_1$-$C_6$ alkoxy group, $C_3$-$C_6$ cycloalkyl group, $C_1$-$C_6$ haloalkyl group, $C_2$-$C_6$ haloalkenyl group, $C_3$-$C_6$ cycloalkyl $C_1$-$C_6$ alkyl group, $C_1$-$C_6$ alkoxy $C_1$-$C_6$ alkyl group, $C_1$-$C_6$ alkylthio $C_1$-$C_6$ alkyl group, -C(=Y)-$R^2$ group, -C(=Y)-$Y^1$-$R^3$ group, -W-C(=O)-$R^4$ group, -W-(C(=O)-$R^4$)$_2$ group, -W-$Y^1$-W-$R^{12}$ group, -W-$Y^1$-(C(=Y))$_m$-$R^{12}$ group, -W-$R^{13}$ group, -$Y^1$-W-$R^{12}$ group, -W-$Y^1$-N=C$R^{12}R^{14}$ group, hydroxy $C_1$-$C_6$ alkyl group, cyano $C_1$-$C_6$ alkyl group, cyanothio $C_1$-$C_6$ alkyl group, isothiocyano $C_1$-$C_6$ alkyl group, nitro $C_1$-$C_6$ alkyl group, $NR^6R^7$ $C_1$-$C_6$ alkyl group, phenyl group (this phenyl group may be substituted with halogen atom, nitro group, cyano group, $C_1$-$C_6$ alkyl group, $C_1$-$C_6$ alkoxy group, $C_1$-$C_6$ haloalkyl group, $C_1$-$C_6$ haloalkoxy group or $NR^6R^7$ group), or $C_3$-$C_{10}$ heterocyclic group having at least one hetero atom which is selected from oxygen atom, sulfur atom and nitrogen atom and which may be the same or different from each other (this heterocyclic group may be substituted with halogen atom, nitro group, cyano group, $C_1$-$C_6$ alkyl group, $C_1$-$C_6$ alkoxy group, $C_1$-$C_6$ haloalkyl group, $C_1$-$C_6$ haloalkoxy group or $NR^6R^7$ group);
X is halogen atom, nitro group, cyano group, $C_1$-$C_6$ alkyl group, $C_1$-$C_6$ alkoxy group, $C_1$-$C_6$ haloalkyl group, $C_1$-$C_6$ haloalkoxy group, or $NR^6R^7$ group;
$R^2$ is $C_1$-$C_6$ alkyl group, $C_1$-$C_6$ alkenyl group, -W-$R^{12}$ group, $C_1$-$C_6$ haloalkyl group, $NR^{15}R^{16}$ group, or phenyl group (this phenyl group may be substituted with halogen atom, nitro group, cyano group, $C_1$-$C_6$ alkyl group, $C_1$-$C_6$ alkoxy group, $C_1$-$C_6$ haloalkyl group, $C_1$-$C_6$ haloalkoxy group or $NR^8R^9$ group);
$R^3$ is $C_1$-$C_{12}$ alkyl group, $C_2$-$C_6$ alkenyl group, $C_2$-$C_6$ alkynyl group, $C_3$-$C_6$ cycloalkyl group, $C_1$-$C_6$ haloalkyl group, $C_1$-$C_6$ alkoxy $C_1$-$C_6$ alkyl group, -W-$R^{12}$ group, benzyl group (this benzyl group may be substituted with halogen atom, nitro group, cyano group, $C_1$-$C_6$ alkyl group, $C_1$-$C_6$ alkoxy group, $C_1$-$C_6$ haloalkyl group, $C_1$-$C_6$ haloalkoxy group or $NR^8R^9$ group), or phenyl group (this phenyl group may be substituted with halogen atom, nitro group, cyano group, $C_1$-$C_6$ alkyl group, $C_1$-$C_6$ alkoxy group, $C_1$-$C_6$ haloalkyl group, $C_1$-$C_6$ haloalkoxy group or $NR^8R^9$ group);
$R^4$ is $C_1$-$C_6$ alkyl group, $C_1$-$C_6$ haloalkyl group, phenyl group (this phenyl group may be substituted with halogen atom, nitro group, cyano group, $C_1$-$C_6$ alkyl group, $C_1$-$C_6$ alkoxy group, $C_1$-$C_6$ haloalkyl group, $C_1$-$C_6$ haloalkoxy

group or $NR^8R^9$ group), $NR^6R^7$ group or $Y^2R^5$ group ($Y^2$ is oxygen atom or sulfur atom);
$R^5$ is $C_2$-$C_6$ alkenyl group, $C_2$-$C_6$ alkynyl group, $C_3$-$C_6$ cycloalkyl group, $C_1$-$C_6$ haloalkyl group, $C_1$-$C_6$ alkoxy $C_1$-$C_6$ alkyl group, benzyl group (this benzyl group may be substituted with halogen atom, nitro group, cyano group, $C_1$-$C_6$ alkyl group, $C_1$-$C_6$ alkoxy group, $C_1$-$C_6$ haloalkyl group, $C_1$-$C_6$ haloalkoxy group or $NR^8R^9$ group), or phenyl group (this phenyl group may be substituted with halogen atom, nitro group, cyano group, $C_1$-$C_6$ alkyl group, $C_1$-$C_6$ alkoxy group, $C_1$-$C_6$ haloalkyl group, $C_1$-$C_6$ haloalkoxy group or $NR^8R^9$ group);
$R^6$ and $R^7$ are each independently hydrogen atom, $C_1$-$C_6$ alkyl group, $C_2$-$C_6$ alkenyl group, -W-$R^{12}$ group, or phenyl group (this phenyl group may be substituted with halogen atom, nitro group, cyano group, $C_1$-$C_6$ alkyl group, $C_1$-$C_6$ alkoxy group, $C_1$-$C_6$ haloalkyl group, $C_1$-$C_6$ haloalkoxy group or $NR^8R^9$ group);
$R^8$ and $R^9$ are each independently hydrogen atom or $C_1$-$C_6$ alkyl group;
$R^{12}$ is phenyl group (this phenyl group may be substituted with halogen atom, nitro group, cyano group, $C_1$-$C_6$ alkyl group, $C_1$-$C_6$ alkoxy group, $C_1$-$C_6$ haloalkyl group, $C_1$-$C_6$ haloalkoxy group or $NR^8R^9$ group), or $C_3$-$C_{10}$ heterocyclic group having at least one hetero atom which is selected from oxygen atom, sulfur atom and nitrogen atom and which may be the same or different from each other (this heterocyclic group may be substituted with halogen atom, nitro group, cyano group, $C_1$-$C_6$ alkyl group, $C_1$-$C_6$ alkoxy group, $C_1$-$C_6$ haloalkyl group, $C_1$-$C_6$ haloalkoxy group or $NR^6R^7$ group);
$R^{13}$ is $C_3$-$C_{10}$ heterocyclic group having at least one hetero atom which is selected from oxygen atom, sulfur atom and nitrogen atom and which may be the same or different from each other (this heterocyclic group may be substituted with halogen atom, nitro group, cyano group, $C_1$-$C_6$ alkyl group, $C_1$-$C_6$ alkoxy group, $C_1$-$C_6$ haloalkyl group, $C_1$-$C_6$ haloalkoxy group or $NR^6R^7$ group);
$R^{14}$ is hydrogen atom or $C_1$-$C_6$ alkyl group;
$R^{15}$ and $R^{16}$ are each independently $C_1$-$C_6$ alkyl group or -W-$R^{13}$ group (when either one of $R^{15}$ and $R^{16}$ is -W-$R^{13}$ group, the other may be hydrogen atom);
n is an integer of 0 to 4;
m is an integer of 0 or 1;
Y is oxygen atom or sulfur atom;
$Y^1$ is oxygen atom or sulfur atom; and
W is $C_1$-$C_4$ alkylene group or $C_1$-$C_4$ alkenylene group}.

**4.** A 1,2-benzisothiazoline-3-thioxo-1,1-dioxide derivative represented by the following general formula [I] or a salt thereof:

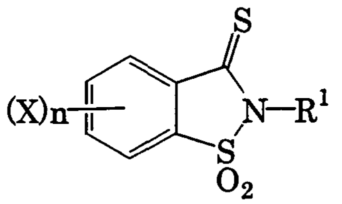

[I]

{wherein $R^1$ is $C_2$-$C_6$ alkenyl group, $C_2$-$C_6$ alkynyl group, $C_1$-$C_6$ alkoxy group, $C_3$-$C_6$ cycloalkyl group, $C_1$-$C_6$ haloalkyl group, $C_2$-$C_6$ haloalkenyl group, $C_3$-$C_6$ cycloalkyl $C_1$-$C_6$ alkyl group, $C_1$-$C_6$ alkoxy $C_1$-$C_6$ alkyl group, $C_1$-$C_6$ alkylthio $C_1$-$C_6$ alkyl group, -C(=Y)-$R^2$ group, -C(=Y)-$Y^1$-$R^3$ group, -W-C(=O)-$R^4$ group, -W-$(C(=O)-R^4)_2$ group, -W-$Y^1$-W-$R^{12}$ group, -W-$Y^1$$(C(=Y))_m$-$R^{12}$ group, -W-$R^{13}$ group, -$Y^1$-W-$R^{12}$ group, -W-$Y^1$-N=C$R^{12}R^{14}$ group, hydroxy $C_1$-$C_6$ alkyl group, cyano $C_1$-$C_6$ alkyl group, cyanothio $C_1$-$C_6$ alkyl group, isothiocyano $C_1$-$C_6$ alkyl group, nitro $C_1$-$C_6$ alkyl group, $NR^6R^7$ $C_1$-$C_6$ alkyl group, phenyl group (this phenyl group may be substituted with halogen atom, nitro group, cyano group, $C_1$-$C_6$ alkyl group, $C_1$-$C_6$ alkoxy group, $C_1$-$C_6$ haloalkyl group, $C_1$-$C_6$ haloalkoxy group or $NR^6R^7$ group), or $C_3$-$C_{10}$ heterocyclic group having at least one hetero atom which is selected from oxygen atom, sulfur atom and nitrogen atom and which may be the same or different from each other (this heterocyclic group may be substituted with halogen atom, nitro group, cyano group, $C_1$-$C_6$ alkyl group, $C_1$-$C_6$ alkoxy group, $C_1$-$C_6$ haloalkyl group, $C_1$-$C_6$ haloalkoxy group or $NR^6R^7$ group);
X is halogen atom, nitro group, cyano group, $C_1$-$C_6$ alkyl group, $C_1$-$C_6$ alkoxy group, $C_1$-$C_6$ haloalkyl group, $C_1$-$C_6$ haloalkoxy group, or $NR^6R^7$ group;
$R^2$ is $C_1$-$C_6$ alkyl group, $C_1$-$C_6$ alkenyl group, -W-$R^{12}$ group, $C_1$-$C_6$ haloalkyl group, or phenyl group (this phenyl group may be substituted with halogen atom, nitro group, cyano group, $C_1$-$C_6$ alkyl group, $C_1$-$C_6$ alkoxy group, $C_1$-$C_6$ haloalkyl group, $C_1$-$C_6$ haloalkoxy group or $NR^8R^9$ group);
$R^3$ is $C_1$-$C_{12}$ alkyl group, $C_2$-$C_6$ alkenyl group, $C_2$-$C_6$ alkynyl group, $C_3$-$C_6$ cycloalkyl group, $C_1$-$C_6$ haloalkyl group, $C_1$-$C_6$ alkoxy $C_1$-$C_6$ alkyl group, -W-$R^{12}$ group, benzyl group (this benzyl group may be substituted with halogen

atom, nitro group, cyano group, $C_1$-$C_6$ alkyl group, $C_1$-$C_6$ alkoxy group, $C_1$-$C_6$ haloalkyl group, $C_1$-$C_6$ haloalkoxy group or $NR^8R^9$ group), or phenyl group (this phenyl group may be substituted with halogen atom, nitro group, cyano group, $C_1$-$C_6$ alkyl group, $C_1$-$C_6$ alkoxy group, $C_1$-$C_6$ haloalkyl group, $C_1$-$C_6$ haloalkoxy group or $NR^8R^9$ group);

$R^4$ is $C_1$-$C_6$ alkyl group, $C_1$-$C_6$ haloalkyl group, phenyl group (this phenyl group may be substituted with halogen atom, nitro group, cyano group, $C_1$-$C_6$ alkyl group, $C_1$-$C_6$ alkoxy group, $C_1$-$C_6$ haloalkyl group, $C_1$-$C_6$ haloalkoxy group or $NR^8R^9$ group), $NR^6R^7$ group or $Y^2R^5$ group ($Y^2$ is oxygen atom or sulfur atom);

$R^5$ is $C_2$-$C_6$ alkenyl group, $C_2$-$C_6$ alkynyl group, $C_3$-$C_6$ cycloalkyl group, $C_1$-$C_6$ haloalkyl group, $C_1$-$C_6$ alkoxy $C_1$-$C_6$ alkyl group, benzyl group (this benzyl group may be substituted with halogen atom, nitro group, cyano group, $C_1$-$C_6$ alkyl group, $C_1$-$C_6$ alkoxy group, $C_1$-$C_6$ haloalkyl group, $C_1$-$C_6$ haloalkoxy group or $NR^8R^9$ group), or phenyl group (this phenyl group may be substituted with halogen atom, nitro group, cyano group, $C_1$-$C_6$ alkyl group, $C_1$-$C_6$ alkoxy group, $C_1$-$C_6$ haloalkyl group, $C_1$-$C_6$ haloalkoxy group or $NR^8R^9$ group);

$R^6$ and $R^7$ are each independently hydrogen atom, $C_1$-$C_6$ alkyl group, $C_2$-$C_6$ alkenyl group, -W-$R^{12}$ group, or phenyl group (this phenyl group may be substituted with halogen atom, nitro group, cyano group, $C_1$-$C_6$ alkyl group, $C_1$-$C_6$ alkoxy group, $C_1$-$C_6$ haloalkyl group, $C_1$-$C_6$ haloalkoxy group or $NR^8R^9$ group);

$R^8$and $R^9$ are each independently hydrogen atom or $C_1$-$C_6$ alkyl group;

$R^{12}$ is phenyl group (this phenyl group may be substituted with halogen atom, nitro group, cyano group, $C_1$-$C_6$ alkyl group, $C_1$-$C_6$ alkoxy group, $C_1$-$C_6$ haloalkyl group, $C_1$-$C_6$ haloalkoxy group or $NR^8R^9$ group), or $C_3$-$C_{10}$ heterocyclic group having at least one hetero atom which is selected from oxygen atom, sulfur atom and nitrogen atom and which may be the same or different from each other (this heterocyclic group may be substituted with halogen atom, nitro group, cyano group, $C_1$-$C_6$ alkyl group, $C_1$-$C_6$ alkoxy group, $C_1$-$C_6$ haloalkyl group, $C_1$-$C_6$ haloalkoxy group or $NR^6R^7$ group);

$R^{13}$ is $C_3$-$C_{10}$ heterocyclic group having at least one hetero atom which is selected from oxygen atom, sulfur atom and nitrogen atom and which may be the same or different from each other (this heterocyclic group may be substituted with halogen atom, nitro group, cyano group, $C_1$-$C_6$ alkyl group, $C_1$-$C_6$ alkoxy group, $C_1$-$C_6$ haloalkyl group, $C_1$-$C_6$ haloalkoxy group or $NR^6R^7$ group);

$R^{14}$ is hydrogen atom or $C_1$-$C_6$ alkyl group;

$R^{15}$ and $R^{16}$ are each independently $C_1$-$C_6$ alkyl group or -W-$R^{13}$ group (when either one of $R^{15}$ and $R^{16}$ is -W-$R^{13}$ group, the other may be hydrogen atom);

n is an integer of 0 to 4;

m is an integer of 0 or 1;

Y is oxygen atom or sulfur atom;

$Y^1$ is oxygen atom or sulfur atom; and

W is $C_1$-$C_4$ alkylene group or $C_1$-$C_4$ alkenylene group}.

**5.** A plant disease control agent for agricultural or horticultural use, containing, as an active ingredient, a 1,2-benzisothiazoline-3-thioxo-1,1-dioxide derivative or a salt thereof, set forth in Claim [3] or [4].

**6.** A pest control agent for agricultural or horticultural use, containing, as an active ingredient, a 1,2-benzisothiazoline-3-thioxo-1,1-dioxide derivative or a salt thereof, set forth in Claim [3] or [4].

**INTERNATIONAL SEARCH REPORT**

International application No. PCT/JP2004/012075

A. CLASSIFICATION OF SUBJECT MATTER
Int.Cl$^7$ A01N43/80, C07D275/06, 417/12

According to International Patent Classification (IPC) or to both national classification and IPC

B. FIELDS SEARCHED

Minimum documentation searched (classification system followed by classification symbols)
Int.Cl$^7$ A01N43/80, C07D275/06, 417/12

Documentation searched other than minimum documentation to the extent that such documents are included in the fields searched

Electronic data base consulted during the international search (name of data base and, where practicable, search terms used)
CA(STN), REGISTRY(STN)

C. DOCUMENTS CONSIDERED TO BE RELEVANT

| Category* | Citation of document, with indication, where appropriate, of the relevant passages | Relevant to claim No. |
|---|---|---|
| Y | US 3699228 A (Nihon Nohyaku Co., Ltd.), 17 October, 1972 (17.10.72), Full text & DE 1953422 A & GB 1278111 A | 1-6 |
| Y | GB 1113634 A (IMPERIAL CHEMICAL INDUSTRIES LTD.), 15 May, 1968 (15.05.68), Full text (Family: none) | 1-6 |
| Y | JP 8-325110 A (Nihon Nohyaku Co., Ltd.), 10 December, 1996 (10.12.96), Full text & WO 96/29871 A2 & EP 141399 A1 & US 6166054 A | 1-6 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed

"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 04 October, 2004 (04.10.04) | 26 October, 2004 (26.10.04) |
| Name and mailing address of the ISA/ Japanese Patent Office | Authorized officer |
| Facsimile No. | Telephone No. |

Form PCT/ISA/210 (second sheet) (January 2004)

**INTERNATIONAL SEARCH REPORT**

International application No. PCT/JP2004/012075

C (Continuation). DOCUMENTS CONSIDERED TO BE RELEVANT

| Category* | Citation of document, with indication, where appropriate, of the relevant passages | Relevant to claim No. |
|---|---|---|
| Y | EP 323869 A1 (SHELL INTERNATIONAL RESEARCH), 12 July, 1989 (12.07.89), Full text (Family: none) | 1-6 |
| Y | JP 54-141764 A (Kumiai Chemical Industry Co., Ltd.), 05 November, 1979 (05.11.79), Full text (Family: none) | 1-6 |
| Y | JP 48-33027 A (Kumiai Chemical Industry Co., Ltd.), 07 May, 1973 (07.05.73), Full text (Family: none) | 1-6 |
| Y | JP 48-10228 A (Kumiai Chemical Industry Co., Ltd.), 08 February, 1973 (08.02.73), Full text (Family: none) | 1-6 |
| Y | JP 48-8930 A (Kumiai Chemical Industry Co., Ltd.), 03 February, 1973 (03.02.73), Full text (Family: none) | 1-6 |
| Y | JP 48-8929 A (Kumiai Chemical Industry Co., Ltd.), 03 February, 1973 (03.02.73), Full text (Family: none) | 1-6 |
| Y | JP 47-43332 A (Kumiai Chemical Industry Co., Ltd.), 19 December, 1972 (19.12.72), Full text (Family: none) | 1-6 |
| Y | JP 47-20158 A (Kumiai Chemical Industry Co., Ltd.), 27 September, 1972 (27.09.72), Full text (Family: none) | 1-6 |
| Y | JP 49-40932 B (Hokko Chemical Industry Co., Ltd.), 06 November, 1974 (06.11.74), Full text (Family: none) | 1-6 |

Form PCT/ISA/210 (continuation of second sheet) (January 2004)

**INTERNATIONAL SEARCH REPORT**

International application No.
PCT/JP2004/012075

C (Continuation). DOCUMENTS CONSIDERED TO BE RELEVANT

| Category* | Citation of document, with indication, where appropriate, of the relevant passages | Relevant to claim No. |
|---|---|---|
| Y | JP 48-35457 B (Kumiai Chemical Industry Co., Ltd.), 27 October, 1973 (27.10.73), Full text (Family: none) | 1-6 |
| Y | JP 48-5907 B (Kumiai Chemical Industry Co., Ltd.), 21 February, 1973 (21.02.73), Full text (Family: none) | 1-6 |
| Y | JP 48-5906 B (Kumiai Chemical Industry Co., Ltd.), 21 February, 1973 (21.02.73), Full text (Family: none) | 1-6 |
| Y | JP 47-420 B (Hokko Chemical Industry Co., Ltd.), 07 January, 1972 (07.01.72), Full text (Family: none) | 1-6 |
| Y | JP 47-419 B (Hokko Chemical Industry Co., Ltd.), 07 January, 1972 (07.01.72), Full text (Family: none) | 1-6 |

Form PCT/ISA/210 (continuation of second sheet) (January 2004)